# EUROPEAN PATENT SPECIFICATION

(11) **EP 3 307 064 B1**
(45) Date of publication and mention of the grant of the patent: **06.09.2023**
(21) Application number: 16808301.2
(22) Date of filing: 09.06.2016
(51) Int. Cl.: A01N 1/00, A01N 1/02, A61P 7/06

(54) **LONG TERM STORAGE AND PRESERVATION OF PLATELETS**
LANGZEITLAGERUNG UND -KONSERVIERUNG VON BLUTPLÄTTCHEN
STOCKAGE DE LONGUE DURÉE ET CONSERVATION DE PLAQUETTES

(30) Priority: 09.06.2015 US 201562173235 P
(43) Date of publication of application: 18.04.2018
(73) Proprietor: President and Fellows of Harvard College, Cambridge, MA 02138 (US); The United States Government as represented by The Department of Veterans Affairs, Washington, DC 20420 (US)
(72) Inventor: THATTE, Hemant, Medfield, MA 02052 (US); SHI, Jialan, Framingham, MA 01701 (US)
(74) Representative: Lahrtz, Fritz
(86) International application number: PCT/US2016/036731
(87) International publication number: WO 2016/201121

(56) References cited:
- WO-A1-2014/106083
- WO-A2-2008/100636
- CN-A- 102 620 950
- CN-A- 103 063 634
- US-A- 4 199 954
- US-B2- 6 743 575
- US-B2- 8 211 628
- US-B2- 8 835 104
- HIROSHI AZUMA ET AL: "Platelet additive solution Electrolytes", TRANSFUSION AND APHERESIS SCIENCE, vol. 44, no. 3, 16 April 2011 (2011-04-16) , pages 277-281, XP028255315, ISSN: 1473-0502, DOI: 10.1016/J.TRANSCI.2011.03.002 [retrieved on 2011-03-30]
- JUNICHI HIRAYAMA ET AL: "Storage of platelets in a novel additive solution (M-sol), which is prepared by mixing solutions approved for clinical use that are not especially for platelet storage", TRANSFUSION., vol. 47, no. 6, 1 June 2007 (2007-06-01), pages 960-965, XP055530811, US ISSN: 0041-1132, DOI: 10.1111/j.1537-2995.2007.01229.x
- HIBA ALHUMAIDAN ET AL: "Current status of additive solutions for platelets", JOURNAL OF CLINICAL APHERESIS, vol. 27, no. 2, 2 February 2012 (2012-02-02), pages 93-98, XP055529398, US ISSN: 0733-2459, DOI: 10.1002/jca.21207
- Jialan Shi, Xiu-Gui Lu and Hemant S Thatte: "Novel Additive Solution "Aayusol" Significantly Preserves Platelets in Lesion-Free State during Extended Storage", BLOOD, vol. 126, no. 23, 3558, 3 December 2015 (2015-12-03), XP002787295, The American Society of Hematology Retrieved from the Internet: URL:http://www.bloodjournal.org/content/12 6/23/3558/tab-article-info [retrieved on 2018-12-10]

## Description

### CROSS-REFERENCE TO RELATED APPLICATIONS

This application claims priority to U.S. Provisional Patent Application No. 62/173,235, filed June 9, 2015.

### FIELD OF INVENTION

This invention relates to compositions for preserving platelets in lesion free functional state and extending the shelf-life of stored human blood platelets.

### BACKGROUND

Platelet transfusions are frequently used to treat patients suffering from massive blood loss or who are undergoing chemotherapy or in case of thrombocytopenia. For example, chemotherapy often reduces the number of platelets, and also causes platelets that are present to function defectively. Storage of platelets for extended periods of time results in the creation of lesion-modified platelets. While significant numbers of platelets can be recovered following storage, the vast majority of them are quickly eliminated from circulation by the spleen and liver due to these storage-mediated lesions. Several approaches for increasing the number of viable platelets recovered after storage have been attempted, such as reduced storage temperature, cryopreservation techniques, additives, and artificial storage media.
Compositions and methods for tissue preservation are disclosed e.g., in WO 2008/100636 A2. Electrolytes as platelet additive solution are disclosed e.g., in Azuma et al., Trans. and Aph. Sci. 2011 44, 277-281. The storage of platelets in a novel additive solution is described e.g., in Hirayama et al., Transfusion 2007, 47(6), 960-965. Alhumaidan and Sweeney in J. Clin. Apher. 2012, 27, 93-98 review additive solutions for platelets.

Despite recent advances in techniques used for platelet storage, the functional capacity and persistence in circulation of platelets recovered by these methods is limited in part due to the continued existence of storage lesions. As such, there continues to be a pressing need for additional methods and compositions for lesion free extended storage of human blood platelets.

### SUMMARY

The present invention is directed to a composition for preserving platelets comprising: a physiological salt solution, glutathione, ascorbic acid, adenosine, dichloroacetate, and less than 1 µM calcium ions wherein the composition has a pH of 7 to 7.7. Provided herein, *inter alia,* are compositions, methods, and kits for decreasing storage-mediated lesions in blood platelets as well as for extending the amount of time that platelets can be stored without attenuated hemostatic function. Also provided herein are compositions, methods, and kits for storing platelets both at ambient temperatures (25 °C) as well as at sub-ambient temperatures ( > 4 to < 25 °C).

In other aspects, also provided herein are methods for preserving platelets, comprising contacting the platelets with any of the compositions disclosed herein. In some embodiments, the platelets produce greater amounts of tonic nitric oxide (NO) compared to platelets that are not contacted with the composition. In some embodiments of any of the embodiments disclosed herein, the method further comprises storing the platelets for 0-15 days. In some embodiments of any of the embodiments disclosed herein, the method further comprises storing the platelets for 0-9 days (such as any of 0, 1, 2, 3, 4, 5, 6, 7, 8, or 9 days). In some embodiments of any of the embodiments disclosed herein, after 1-9 days of storage, the platelets do not exhibit aggregation. In some embodiments of any of the embodiments disclosed herein, after 1-8 days of storage, the platelets produce greater amounts of high energy phosphates compared to platelets that are not contacted with the composition. In some embodiments of any of the embodiments disclosed herein, after 1-9 days of storage, the platelets exhibit less apoptosis compared to platelets that are not contacted with the composition. In some embodiments of any of the embodiments disclosed herein, after 1-9 days of storage, the platelets exhibit less activation (P-Selectin expression) compared to platelets that are not contacted with the composition. In some embodiments of any of the embodiments disclosed herein, after 1-9 days of storage, the platelets exhibit decreased loss of discoid morphology compared to platelets that are not contacted with the composition. In some embodiments of any of the embodiments disclosed herein, the platelets are stored at ambient or room temperature. In some embodiments of any of the embodiments disclosed herein, the platelets are stored at 21 ± 2 °C. In some embodiments of any of the embodiments disclosed herein, the platelets are stored at 4 ± 1 °C or at 13± 3 °C. In some embodiments of any of the embodiments disclosed herein, the platelets are stored at about 3 °C to about 18 °C.

In yet other aspects, provided herein are methods for detecting the presence of nitric oxide in platelets in real time, the method comprising: (a) labeling platelets with a nitric oxide-specific fluorescent dye; (b) rapidly fixing the platelets; and (c) detecting the presence of the fluorescent dye. In some embodiments the platelets have been in storage for 0-9 days. In some embodiments of any of the embodiments disclosed herein, the platelets have been stored in any of the compositions disclosed herein. In some embodiments of any of the embodiments disclosed herein, the nitric oxide-specific fluorescent dye is diaminofluorescein acetate (DAF2-DA) or a derivative of diaminofluorescein (DAF-2) (such as, diaminofluorescein ester;diaminofluorescein-FM (DAF-FM) or diaminofluorescein-FMdiacetate (DAF-FM diacetate). In some embodiments of any of the embodiments disclosed herein, the platelets are fixed with gluteraldehyde. In some embodiments of any of the embodiments disclosed herein, the presence of the fluorescent dye is detected with confocal microscopy, multiphoton microscopy and fluorescence activated cell sorting (FACS) scanning/analysis.

Each of the aspects and embodiments described herein are capable of being used together, unless excluded either explicitly or clearly from the context of the embodiment or aspect.

The gradual loss of quality in stored platelets as measured collectively with various standard metabolic, functional, and morphologic *in vitro* assays is known as the platelet storage lesion. The biochemical, structural and functional changes that occur during platelet storage under blood bank conditions impact platelet viability and hemostatic function. Platelet storage lesion (PSL) is associated with morphological changes and platelet activation followed by microvesciculation and loss of function, leading to transfusion failure. Such deleterious changes in structure and function can restrict the platelet shelf life to 5 days or fewer.

Throughout this specification, various patents, patent applications and other types of publications (e.g., journal articles, electronic database entries, etc.) are referenced.

### BRIEF DESCRIPTION OF THE DRAWINGS

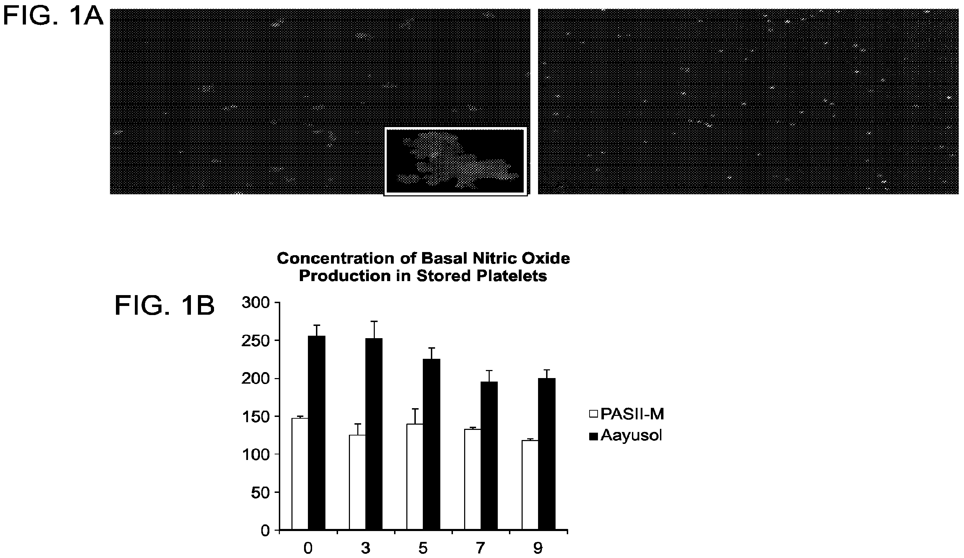

FIG. 1A is a photograph and FIG 1B is a bar graph depicting platelets labeled periodically with DAF2-DA, a nitric oxide-specific fluorescence dye and imaged using confocal microscopy. Representative image of n=3 experiments; 200X magnification. Images show that after day 5 (x-axis) of storage, platelets in Aayusol were producing greater amount of tonic nitric oxide as indicated by the brightness of green fluorescence (y-axis) as compared to those in PASIII-M.

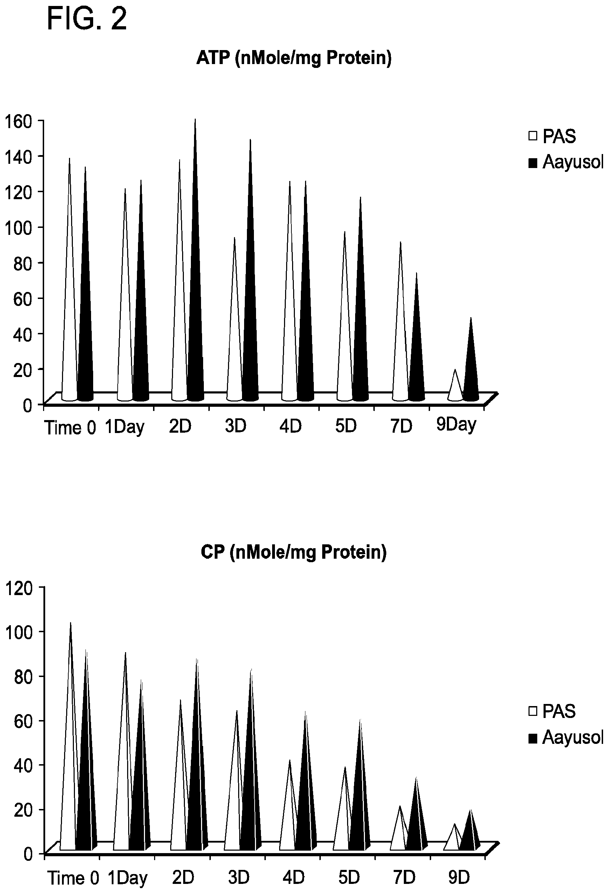

FIG. 2 depicts high energy phosphate concentrations in platelets during extended storage. Platelet concentrates were stored in Aayusol or PASIII-M (20:80) for 15 days at ambient temperature (21± 2°C) with gentle shaking. PRP (Platelet rich plasma) was used as a control. Platelets were periodically evaluated for ATP concentration (n =3). CP = creatine phosphate.

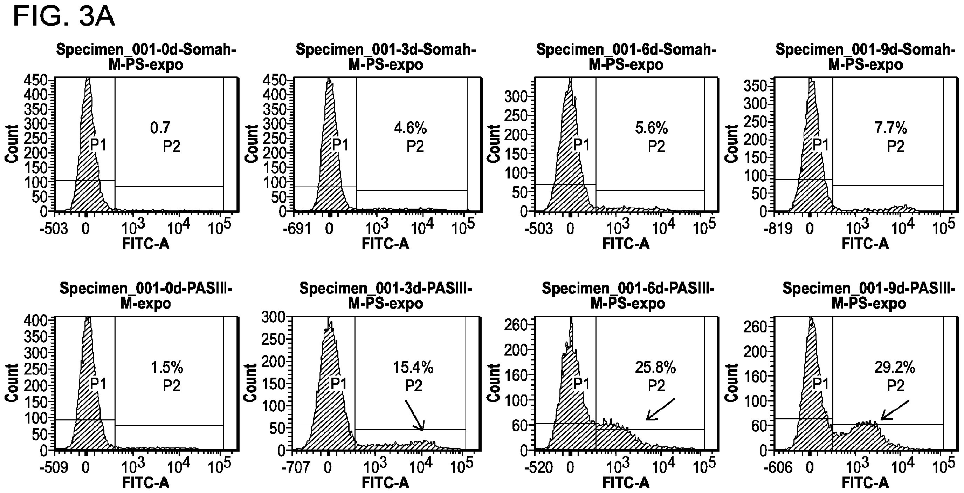

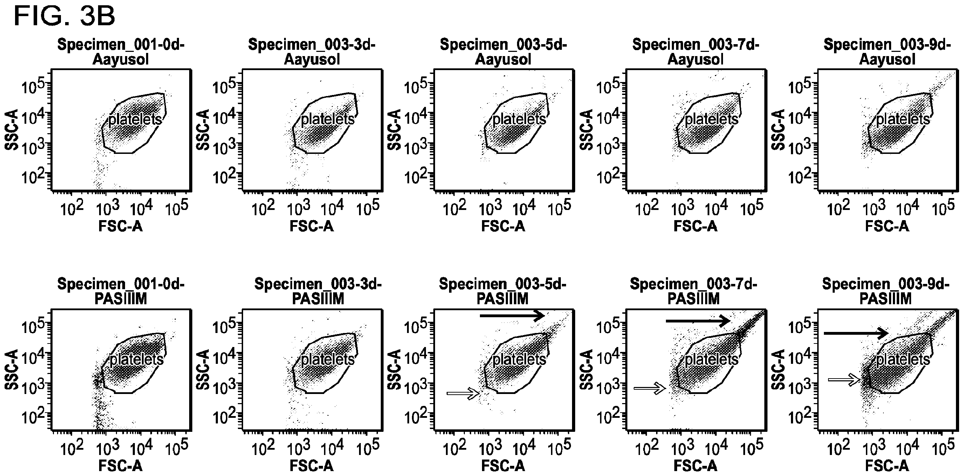

FIG. 3A and FIG. 3B are graphs depicting flow-cytometric analysis of phosphatidylserine (PS) exposure in stored platelets. Platelets were stored in either Aayusol (top row) or PASIIIM (bottom row) at ambient (21± 2°C) temperature for 0, 3, 6, and 9 days and incubated with FITC-lactadherin in the dark for 10 min at room temperature before flow cytometric analysis. Samples were analyzed using wavelengths of 488 nm (excitation) and 530 nm (emission), respectively. Representative image of three independent experiments.

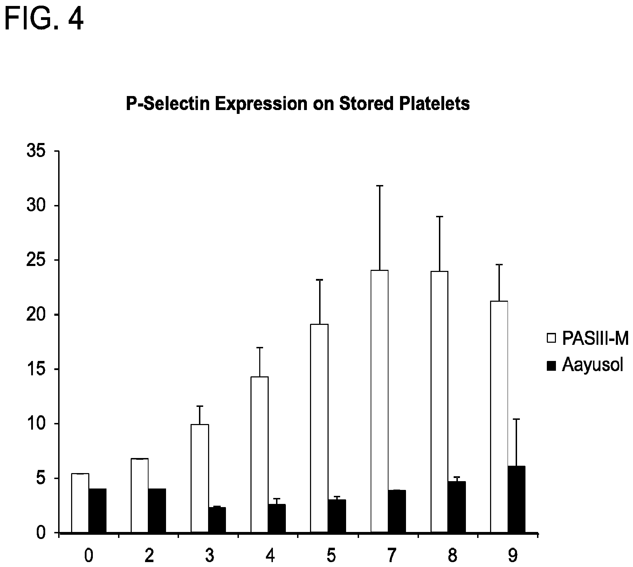

FIG. 4 is a bar graph depicting flow-cytometric analysis of P-Selectin expression on platelets during storage. Platelets were stored in Aayusol at ambient (21± 2°C) temperature for 0, 3, 5, and 9 days and incubated with FITC-anti P-Selectin (CD62) antibody in the dark for 10 min at room temperature before flow cytometric analysis. Samples were analyzed using wavelengths of 488 nm (excitation) and 530 nm (emission), respectively. Representative image of three independent experiments showing extent platelet activation. P-selectin expression on platelets stored in Aayusol was significantly lower than those stored PASIII-M indicating attenuated activation during storage.

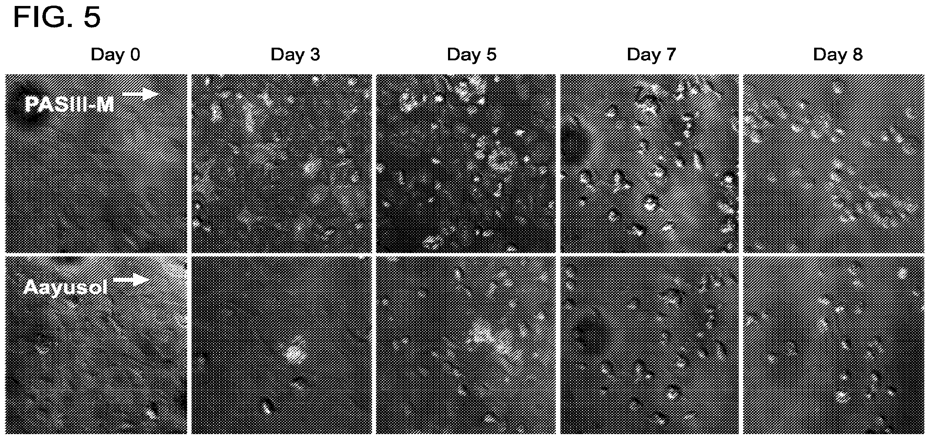

FIG. 5 is a photograph depicting confocal microscopy of stored platelets. Platelets were stored in PASIII-M and Aayusol for 0-9 days and were periodically labeled with FITC-lactadherin to identify PS exposure (induction of apoptosis) and assess morphology. Cells were imaged using Zeiss LSM 710 confocal microscope using 63X oil immersion objective. Platelets showed progressive increase in PS exposure.

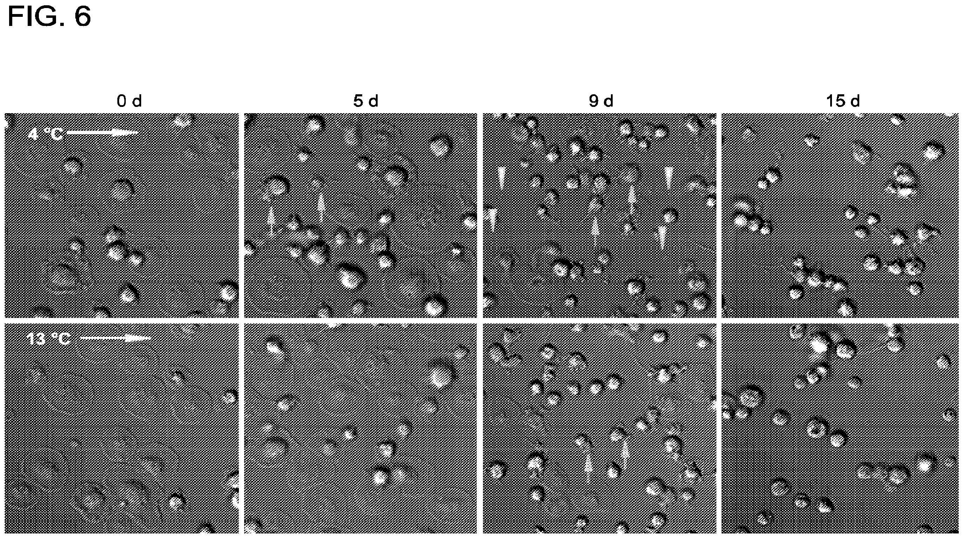

FIG. 6 is a photomicrograph depicting the morphology of platelets stored at sub-ambient temperatures. Platelets stored in Aayusol at 4 ± 1 °C and 13 ± 3 °C were imaged on 0, 5, 9 and 15 days using a confocal microscope.

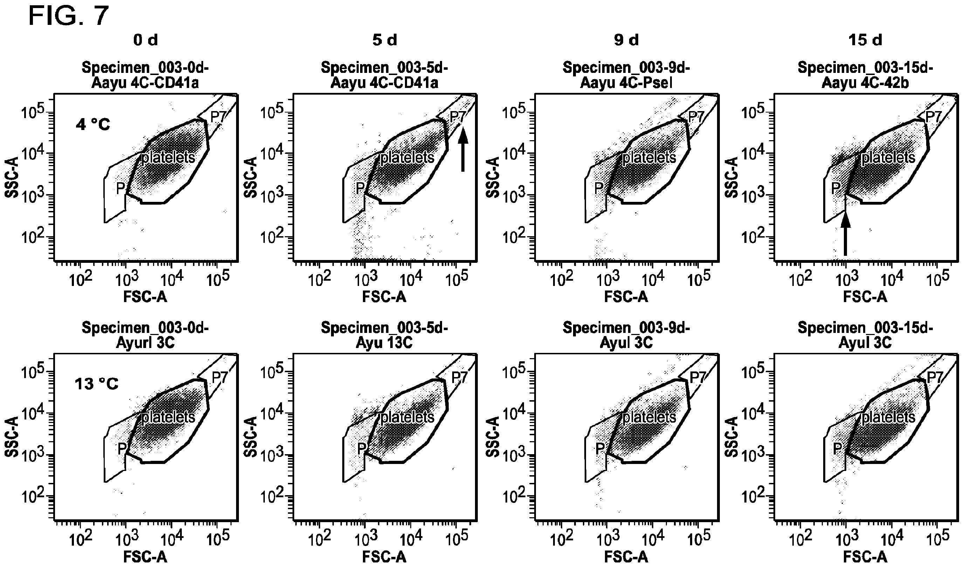

FIG. 7 is a dot plot depicting qualitative and quantitative analyses of platelet aggregates and microparticles (MPs) in stored platelets. A representative set of dot plots show platelets, aggregates, and MPs stored at 4 °C (upper panel) and at 13 °C (lower panel). There was a progressive but non-significant increase in microparticles (P; green) and aggregates (P7; blue) formation during storage, (greater at 4°C), however, the distribution of platelet population (red) at both the temperatures remained consistent during storage.

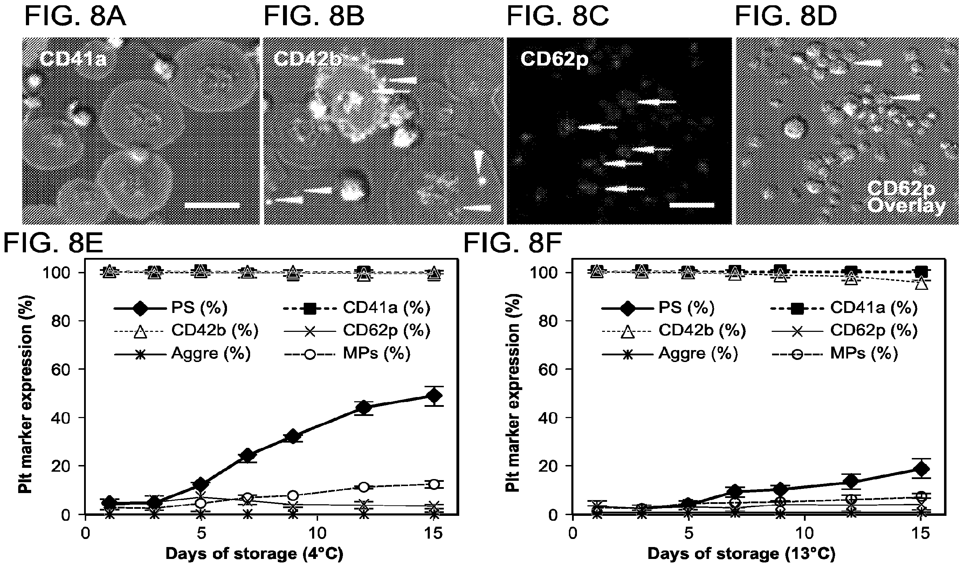

FIG. 8 is a photomicrograph depicting morphology (upper panel) and a graph showing a quantitative analyses of platelet markers (lower panel) on platelets stored in Ayausol at sub ambient temperatures. Platelets were sampled periodically at 0, 3, 5, 7, 9, 12 and 15 days and labeled with fluorescently tagged anti - CD 41a, CD 42b, CD62p (p-selectin) antibodies. PS was labeled with FITC-lactadherin. Samples were imaged with fluorescence confocal microscopy (upper panels) and quantitative flow cytometry (lower panels); n =5.

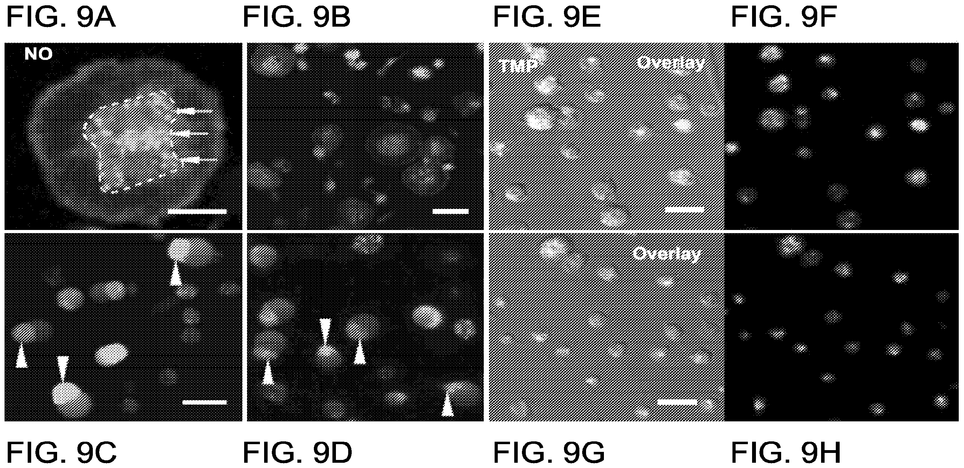

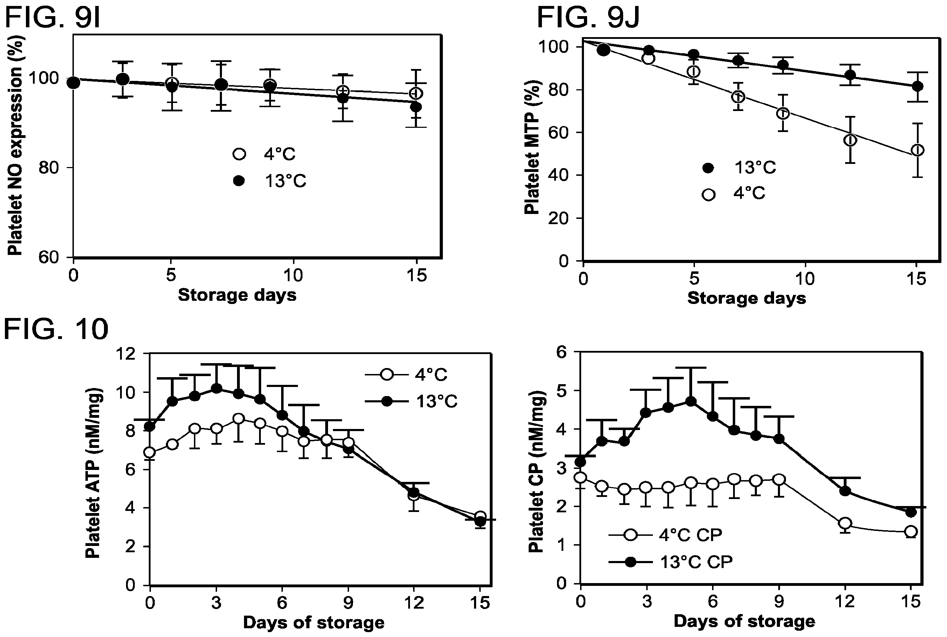

FIG. 9 is a photomicrograph depicting NO production in stored platelets (upper panel) and a graph showing a quantitative analysis of NO production (lowed panel). NO synthesis was visualized with the cell permeable fluorescent precursor, 4-amino-5-methylamino-2',7'-difluorofluorescein (DAF-FM) diacetate on 0, 5, 9, and 15 days by confocal microscope (upper panel). Additionally, the NO synthesis in the stored platelets was also quantified by flow cytometer from the fluorescence distribution plot of the platelets loaded with DAF-FM (lower panel). Representative data are from 5 experiments.
FIG. 10 depicts graphs showing high-energy phosphate (HEP) levels in platelets stored at sub-ambient temperatures in Aayusol. Each bar represents mean ± SEM of n = 5 for each group.

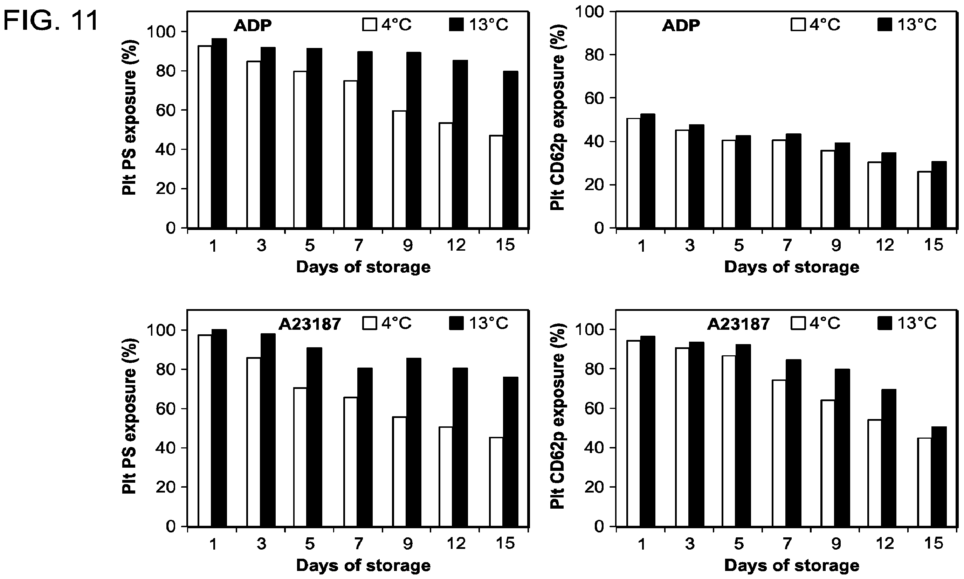

FIG. 11 depicts graphs showing that Platelets stored in Aayusol at sub-ambient temperatures remain functionally active upon agonist stimulation. The stored platelets were stimulated with 50 µM ADP and/or 2 µM A23187/ on 0, 1, 3, 5, 7, 9, 12 and 15 days, were stained with FITC-lactadherin (a PS probe) and Alexa 647 anti-CD62p Ab, and were analyzed by flow cytometry. Results from 2 independent experiments.

### DETAILED DESCRIPTION

The gradual loss of quality in stored platelets as measured collectively with various standard metabolic, functional, and morphologic *in vitro* assays is known as the platelet storage lesion. The biochemical, structural and functional changes that occur during platelet storage under blood bank conditions impact platelet viability and hemostatic function. Platelet storage lesion (PSL) is associated with morphological changes and platelet activation followed by microvesciculation and loss of function, leading to transfusion failure. Such deleterious changes in structure and function can restrict the platelet shelf life to 5 days or fewer.

The invention described herein provides, *inter alia,* compositions for preserving platelets in a lesion-free and functional state during extended storage periods of up to 15 days. Described are methods and kits for utilizing the same. In contrast to currently available compositions and techniques for preserving platelets, platelets stored in the compositions disclosed herein are characterized by a marked decrease in "storage-lesions," which are associated with attenuated hemostatic function. During platelet storage, the compositions disclosed herein (1) maintain and/or restore platelet energy states and morphology; (2) maintain active metabolism and buffering; (3) preserve and/or promote nitric oxide production; and (4) prevent microparticle formation, aggregation and activation during storage. As such, long term preservation in the compositions described herein result in platelets that are fully functional and lesion-free. Consequently, the compositions and methods disclosed herein have the potential to allow platelets to be stored for longer periods of time compared to what has previously been possible at ambient temperatures. The invention described herein will therefore help alleviate continual platelet shortages, particularly in remote areas with small populations, as well as help to decrease healthcare costs by attenuating patient morbidity. Moreover, the platelet storage compositions described herein result in decreased allergic transfusion reactions (ATR) in individuals receiving platelets stored in these solutions because of reduced plasma concentration. Furthermore, the compositions disclosed herein can serve as a blood volume expander/replacement agent or as a resuscitation fluid in subjects who have suffered sudden massive blood loss or who are undergoing chemotherapy. The solution will also provide an energy source as well as a substrate for vasodilation.

The combination of carnosine and carnitine synergistically produces a higher amount of high energy phosphates in platelets stored in any of the solutions disclosed herein compared to the amount of HEPs produced using a storage solution lacking these ingredients. In another embodiment, the combination of carnosine, carnitine, glucose, and creatine synergistically produces a higher amount of high energy phosphates in platelets stored in any of the solutions disclosed herein compared to the amount of HEPs produced using a storage solution lacking these ingredients. In other embodiments, the combination of citrulline and arginine synergistically produces a higher amount of nitrous oxide (NO) in platelets stored in any of the solutions disclosed herein compared to the amount of NO produced using a storage solution lacking these ingredients. The synergism demonstrated by the combination of these components is both unexpected and surprising.

Additionally, the invention described herein provides compositions and disclosed are methods for preserving platelets in a lesion-free and functional state during extended storage periods at both ambient (*i.e*. room) temperatures as well as at sub-ambient (*i.e.* between about 3 to about 18 °C) temperatures. Previously available compositions and methods for preserving platelets required that they be stored only at ambient temperatures to prevent damage which results in rapid clearance by the body once administered to a patient. Thus, the compositions and methods provided herein not only allow platelets to be stored for vastly longer periods of time than what was previously attainable but also permits storage of platelets over a much broader range of temperatures compared to what was previously possible.

### 1. Definitions

As used herein, the term "physiological salt" refers to any salt which, when in aqueous solution at a given concentration, assists with or is required for a cellular or physiologic function. Examples of physiological salts include, without limitation, alkaline and alkaline earth metal chlorides, phosphates and sulfates, such as, KCl, NaCl, MgCl₂, MgSO₄, and mixtures thereof.

The phrase "platelet storage lesion" or variants of the same as used herein include all of the physiological, biochemical, and morphological changes that accompany the storage of platelets. These include, without limitation, one or more of rearrangement of the platelet cytoskeleton, microvesiculation, translocation of phosphatidylserine to the outer leaflet of the plasma membrane, aggregation, activation, and changes in the surface expression of various adhesive platelet glycoproteins, including CD62P (P-selectin) and CD42b (GPIbc). In some embodiments, platelet storage lesions occur during processing and/or storage of platelets subsequent to any of mechanical trauma, hypoxic conditions and/or exposure to cold.

A "subject" can be a vertebrate, a mammal, or a human. Mammals include, but are not limited to, farm animals, sport animals, pets, primates, mice and rats. In one aspect, a subject is a human.

As used herein, "ambient temperature" or "room temperature" is any temperature in the range of about 21 ± 2°C, such as any of about 19 °C, 20 °C, 21 °C, 22 °C, or 23 °C. " Sub-ambient temperatures," as used herein, refers to temperatures from about 0 °C to about 18 °C, such as any of about 0 °C, 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, or 18 °C.

Unless defined otherwise herein, all technical and scientific terms used herein have the same meaning as commonly understood by one of ordinary skill in the art to which this invention pertains.

As used herein, the singular terms "a," "an," and "the" include the plural reference unless the context clearly indicates otherwise.

The transitional term "comprising," which is synonymous with "including," "containing," or "characterized by," is inclusive or open-ended and does not exclude additional, unrecited elements or method steps. By contrast, the transitional phrase "consisting of excludes any element, step, or ingredient not specified in the claim. The transitional phrase "consisting essentially of limits the scope of a claim to the specified materials or steps "and those that do not materially affect the basic and novel characteristic(s)" of the claimed invention

### II. Compositions of the Invention

Platelets are anucleate bone marrow-derived blood cells that protect injured mammals from blood loss by adhering to sites of vascular injury and by promoting the formation of plasma fibrin clots. Humans depleted of circulating platelets, for example, by bone marrow failure suffer from life threatening spontaneous bleeding and less severe deficiencies of platelets contribute to bleeding complications following trauma or surgery.

A reduction in the number of circulating platelets to below ~70,000 per µL results in the prolongation of a standardized cutaneous bleeding time test, extrapolating to near infinity as the platelet count falls to zero (thrombocytopenia). Subjects with platelet counts of less than 20,000 per µL are thought to be highly susceptible to spontaneous hemorrhage from mucosal surfaces, especially when the thrombocytopenia is caused by bone marrow failure, chemotherapy, or traumatic injury resulting in massive blood loss. The platelet deficiencies associated with bone marrow disorders such as aplastic anemia, acute and chronic leukemia, metastatic cancer but especially resulting from cancer treatment with ionizing radiation and chemotherapy represent a major public health problem. Thrombocytopenia associated with major surgery, injury and sepsis also eventuates in administration of significant numbers of platelet transfusions.

A major advance in medical care half a century ago was the development of platelet transfusions to correct such platelet deficiencies, and over 9 million platelet transfusions took place in the United States alone in 1999 (Jacobs, J Am Geriatr Soc. 2001 Jan;49(1):91-4.). Platelets, however, unlike all other transplantable tissues, do not tolerate refrigeration, because they disappear rapidly from the circulation of recipients if subjected to even very short periods of chilling, and the cooling effect that shortens platelet survival is irreversible (Berger et al., Blood. 1998 Dec 1;92(11):4446-52.).

The resulting need to keep these cells at room temperature prior to transfusion has imposed a unique set of costly and complex logistical requirements for platelet storage. Because platelets are actively metabolic at room temperature, they require constant agitation in porous containers to allow for release of evolved CO₂ to prevent the toxic consequences of metabolic acidosis. Room temperature storage conditions result in macromolecular degradation and reduced hemostatic functions of platelets, a set of defects known as "storage lesions." Platelets with storage lesions are rapidly cleared from a subject's primary circulation by the spleen and are therefore unsuitable for use in those subjects in dire need of replenishment of these cells.

Currently available techniques and compositions for storage of platelets permit only around 3-5 days of storage prior to the onset of significant storage lesions.

The compositions of the present invention are platelet additive solutions for preserving platelets in a lesion free and functional state during extended storage, both at ambient as well as at sub-ambient temperatures. Platelets can be stored in the compositions described herein for up to 15 days, such as any of 1, 2, 3, 4, 5, 6, 7, 8, 9, 10, 11, 12, 13, 14, or 15 days, without significant accumulation of storage lesions, without significant decreases in nitric oxide synthesis, without significant decreases in high energy phosphate concentration, without significant indications of an apoptotic state (for example, phosphatidylserine (PS) translocation to the outer cellular membrane) and with significantly decreased levels of aggregation and morphological defects.

### A. Physiological Salt Solutions

The compositions of the present invention can be aqueous (*i.e*. water-based) solutions that include a physiological salt solution, glucose, glutathione, ascorbic acid, arginine, citrulline malate, adenosine, creatine, and less than 1 µM of a calcium ion source (for example, calcium chloride). Dichloroacetate must be present. The physiological salt solution can include any salt which, when in aqueous solution at a given concentration, assists or is required for a physiologic function such as maintaining ionic concentrations (for example, potassium, magnesium, sodium, chloride, sulfate, phosphate, and bicarbonate ionic concentrations) inside and outside of stored platelets as well as controlling the amount of water that can traverse the platelet cellular membrane. The components of the physiological salt solution can also help to buffer and maintain a proper pH in the platelet storage solution. Particular salts capable of use in the present in invention include, without limitation, potassium chloride, potassium phosphate, magnesium chloride, magnesium sulfate, sodium chloride, sodium bicarbonate and sodium phosphate.

In some embodiments of the compositions disclosed herein, the physiological salt solution contains a potassium ion source, such as, potassium chloride. The concentration of potassium chloride in the platelet storage composition can be between about 4-5 mM, such as about 4 mM or about 5 mM. In another embodiment, the platelet storage composition can contain about 0.30 g/L potassium chloride.

The physiological salt solution of any of the compositions disclosed herein can contain a sodium ion source. Sodium ions can be added to the physiological salt solution in the form of a sodium salt, such as one or more sodium salts selected from the group consisting of NaAlO₂, NaBO₂, NaCl, NaClO, NaClO₂, NaClO₃, NaClO₄, NaF, Na₂FeO₄, NaHCO₃, NaH₂PO₄, NaHSO₃, NaHSO₄, NaI, NaMnO₄, NaNH₂, NaNO₂, NaNO₃, NaOH, NaPO₂H₂, NaSH, Na₂MnO₄, Na₃MnO₄, Na₂N₂O₂, Na₃O₂, Na2SO₃, Na₂SO₄, Na₂S₂O₄, Na₂SeO₃, Na₂SeO₄, Na₂SiO₃, Na₂Si₂O₅, Na4SiO₄, Na₂Ti₃O₇, Na₂Zn(OH)₄, NaH₂C₆H₅O₇, and Na₃PO₄. In some embodiments, sodium ions in the platelet storage composition be at a concentration of between about 100-140 mM, such as about 100 mM, about 101 mM, about 102 mM, about 103 mM, about 104 mM, about 105 mM, about 106 mM, about 107 mM, about 108 mM, about 109 mM, about 110 mM, about 111 mM, about 112 mM, about 113 mM, about 114 mM, about 115 mM, about 116 mM, about 117 mM, about 118 mM, about 119 mM, about 120 mM, about 121 mM, about 122 mM, about 123 mM, about 124 mM, about 125 mM, about 126 mM, about 127 mM, about 128 mM, about 129 mM, about 130 mM, about 131 mM, about 132 mM, about 133 mM, about 134 mM, about 135 mM, about 136 mM, about 137 mM, about 138 mM, about 139 mM, or about 140 mM including all ranges and numbers falling within these values.

In other embodiments of the compositions disclosed herein, the physiological salt solution contains sodium chloride. The concentration of sodium chloride in the platelet storage composition can be between about 100-115 mM, such as about 100 mM, about 101 mM, about 102 mM, about 103 mM, about 104 mM, about 105 mM, about 106 mM, about 107 mM, about 108 mM, about 109 mM, about 110 mM, about 111 mM, about 112 mM, about 113 mM, about 114 mM, or about 115 mM, including all ranges and numbers falling within these values. In another embodiment, the platelet storage composition can contain about 5.84 g/L sodium chloride.

In further embodiments of the compositions disclosed herein, the physiological salt solution contains sodium phosphate. The concentration of sodium phosphate in the platelet storage composition can be between about 10-40 mM, such as about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM, about 30 mM, about 31 mM, about 32 mM, about 33 mM, about 34 mM, about 35 mM, about 36 mM, about 37 mM, about 38 mM, about 39 mM, or about 40 mM, including all ranges and numbers falling within these values. In another embodiment, the platelet storage composition can contain about 7.9 g/L sodium phosphate. Any form of sodium phosphate can be used in the present invention, including, without limitation, the dibasic heptahydrate form.

In further embodiments of the compositions disclosed herein, the physiological salt solution contains sodium citrate. The concentration of sodium citrate in the platelet storage composition can be between about 5-15 mM, such as about 5 mM, about 6 mM, about 7 mM, about 8 mM, about 9 mM, about 10 mM, about 11 mM, about 12 mM, about 13 mM, about 14 mM, or about 15 mM including all ranges and numbers falling within these values. In another embodiment, the platelet storage composition can contain about 4.41 g/L sodium citrate.

In another embodiment of the compositions disclosed herein, the physiological salt solution contains sodium bicarbonate. The concentration of sodium bicarbonate in the platelet storage composition can be between about 4-6 mM, such as about 4.5 mM, 5 mM, 5.5 mM, or 6 mM, including all ranges and numbers falling within these values. In one embodiment, the concentration of magnesium sulfate is about 5 mM. In another embodiment, the platelet storage composition can contain about 0.42 g/L sodium bicarbonate.

In another embodiment of the compositions disclosed herein, the physiological salt solution contains no calcium ions (for example, calcium ions supplied by calcium salts such as calcium chloride) or, alternatively, contains less than 1 such as less than about 0.1 µM, about 0.01 µM, about 0.001 µM, or about 0.0001 µM, including all ranges and numbers falling within these values. In another embodiment of the compositions disclosed herein, the physiological salt solution contains no calcium ions (for example, calcium ions supplied by calcium salts such as calcium chloride) or, alternatively, contains less than 1 µM calcium ions. In another embodiment, the physiological salt solution of the compositions disclosed herein contain less than 1 µM calcium ions supplied from one or more calcium salts such as those selected from the group consisting of calcium acetate, calcium aluminates, calcium aluminoferrite, calcium aluminosilicate, calcium ammonium nitrate, calcium arsenate, calcium ascorbate, calcium azide, calcium benzoate, calcium beta-hydroxy-beta-methylbutyrate, calcium bicarbonate, calcium bisulfite, calcium borate, calcium bromate, calcium bromide, calcium carbide, calcium carbonate, calcium chlorate, calcium chromate, calcium citrate, calcium citrate malate, calcium copper titanate, calcium cyanamide, calcium diglutamate, calcium erythorbate, calcium fluoride, calcium formate, calcium fumarate, calcium glubionate, calcium glucoheptonate, calcium gluconate, calcium glycerylphosphate, calcium guanylate, calcium hexaboride, calcium hydride, calcium hydroxide, calcium hypochlorite, calcium inosinate, calcium iodate, calcium iodide, calcium lactate, calcium lactate gluconate, calcium magnesium acetate, calcium malate, calcium monohydride, calcium monophosphide, calcium morphenate, calcium nitrate, calcium nitride, calcium nitrite, calcium oxalate, calcium oxide, calcium pangamate, calcium perchlorate, calcium permanganate, calcium peroxide, calcium phosphate, calcium phosphide, calcium propanoate, calcium pyrophosphate, calcium silicate, calcium silicate hydrate, calcium silicide, calcium sorbate, calcium stearate, calcium sulfate, calcium sulfate, calcium sulfide, calcium sulfite, calcium tartrate, calcium titanate, calcium chloride, and calcium cyanide.

The physiological salt solution of any of the compositions disclosed herein (such as physiological salt solutions used in compositions for platelet storage at both room/ambient temperatures (21 ± 2°C) or sub-ambient temperatures (4 ± 1°C and 13 ±3°C) may further comprise one or more calcium chelator compounds. Non-limiting examples of calcium chelators available for use in the present invention include one or more of ethylenediaminetetraacetic acid (EDTA), ethyleneglycoltetraacetic acid (EGTA) (1.5-5.0 mM), diethylenetriaminepentaacetate (DTPA), hydroxyethylethylenediaminetriacetic acid (HEEDTA), diaminocyclohexanetetraacetic acid (CDTA), 1,2-bis(2-aminophenoxy)ethane-N,N,N',N'-tetraacetic acid (BAPTA), citric acid, and pharmaceutically acceptable salts thereof. In one embodiment, the physiological salt solution of any of the compositions disclosed herein contains 0.001 - 5 mM calcium chelator (*e.g.,* EGTA), such as any of 0.001 mM, 0.01 mM, 0.1 mM, 0.2 mM, 0.3 mM, 0.4 mM, 0.5 mM, 0.6 mM, 0.7 mM, 0.8 mM, 0.9 mM, 1 mM, 1.1 mM, 1.2 mM, 1.3 mM, 1.4 mM, 1.5 mM, 1.6 mM, 1.7 mM, 1.8 mM, 1.9 mM, 2 mM, 2.1 mM, 2.2 mM, 2.3 mM, 2.4 mM, 2.5 mM, 2.6 mM, 2.7 mM, 2.8 mM, 2.9 mM, 3 mM, 3.1 mM, 3.2 mM, 3.3 mM, 3.4 mM, 3.5 mM, 3.6 mM, 3.7 mM, 3.8 mM, 3.9 mM, 4 mM, 4.1 mM, 4.2 mM, 4.3 mM, 4.4 mM, 4.5 mM, 4.6 mM, 4.7 mM, 4.8 mM, 4.9 mM, or 5 mM calcium chelator.

The physiological salt solution of any of the compositions disclosed herein can contain a potassium ion source. Potassium ions can be added to the physiological salt solution in the form of a potassium salt, such as one or more potassium salts selected from the group consisting of KAsO₂, KBr, KBrO₃, KCN, KCNO, KCl, KClO₃, KClO₄, KF, KH, KHCO₂, KHCO₃, KHF₂, KHS, KHSO₃, KHSO₄, KH₂AsO₄, KH₂PO₃, KH₂PO_{4,} KI, KIO₃, KIO₄, KMnO₄, KN₃, KNH₂, KNO₂, KNO₃, KOCN, KOH, KO₂, KPF₆, KCH₃COO, K₂Al₂O₄, K₂CO₃, K₂CrO₄, K₂Cr₂O₇, K₂FeO₄, K₂HPO₄, K₂MnO₄, K₂O, K₂O₂, K₂S, K₂SeO₄, K₂SO₃, K₂SO₄, KHSO₅, K₂S₂O₅, K₂S₂O₇, K₂S₂O₈, K₂SiO₃, K₃[Fe(C₂O₄)₃], K₄[Fe(CN)₆], K₃PO₄, and K₄Mo₂Cl₈. In some embodiments, potassium ions in the platelet storage composition be at a concentration of between about 0.01-1 mM, such as about 0.1 mM, about 0.15 mM, about 0.2 mM, about 0.25 mM, about 0.3 mM, about 0.35 mM, about 0.4 mM, about 0.45 mM, about 0.5 mM, about 0.55 mM, about 0.6 mM, about 0.65 mM, about 0.7 mM, about 0.75 mM, about 0.8 mM, about 0.85 mM, about 0.9 mM, or about 1 mM including all ranges and numbers falling within these values.

In other embodiments of the compositions disclosed herein, the physiological salt solution contains potassium phosphate. The concentration of potassium phosphate in the platelet storage composition can be between about 0.3-0.5 mM, such as about 0.35 mM, 0.4 mM, 0.45 mM, or 0.5 mM, including all ranges and numbers falling within these values. In one embodiment, the concentration of potassium phosphate is about 0.44 mM. In another embodiment, the platelet storage composition can contain about 0.06 g/L potassium phosphate. Any form of potassium phosphate can be used in the present invention, including, without limitation, the monobasic form.

The physiological salt solution of any of the compositions disclosed herein can contain a magnesium ion source. Magnesium ions can be added to the physiological salt solution in the form of a magnesium salt, such as one or more magnesium salts selected from the group consisting of MgB₂, MgBr₂, MgCO₃, MgC₂O₄, MgC₆H₆O₇, MgC₁₄H₁₀O₄, MgCl₂, Mg(ClO₄)₂, MgF₂, MgH₂, Mg(HCO₃)₂, MgI₂, Mg(NO₃)₂, MgO, MgO₂, Mg(OH)₂, MgS, MgSO₃, MgSO₄, Mg₂Al₃, Mg₂Si, Mg₂SiO₄, Mg₂Si₃O₈, Mg₃N₂, Mg₃(PO₄)₂, and Mg₂(CrO₄)₂. In some embodiments, magnesium ions in the platelet storage composition be at a concentration of between about 0.01-1.5 mM, such as about 0.1 mM, about 0.15 mM, about 0.2 mM, about 0.25 mM, about 0.3 mM, about 0.35 mM, about 0.4 mM, about 0.45 mM, about 0.5 mM, about 0.55 mM, about 0.6 mM, about 0.65 mM, about 0.7 mM, about 0.75 mM, about 0.8 mM, about 0.85 mM, about 0.9 mM, about 1 mM, about 1.05 mM, about 1.1 mM, about 1.15 mM, about 1.2 mM, about 1.25 mM, about 1.3 mM, about 1.35 mM, about 1.4 mM, about 1.45 mM, or about 1.5 mM including all ranges and numbers falling within these values.

In still further embodiments of the compositions disclosed herein, the physiological salt solution contains magnesium chloride. The concentration of magnesium chloride in the platelet storage composition can be between about 0.4-0.6 mM, such as about 0.45 mM, 0.5 mM, 0.55 mM, or 0.6 mM, including all ranges and numbers falling within these values. In one embodiment, the concentration of magnesium chloride is about 0.5 mM. In another embodiment, the platelet storage composition can contain about 0.110 g/L magnesium chloride. Any form of magnesium chloride can be used in the present invention, including, without limitation, the hexahydrate form.

In other embodiments of the compositions disclosed herein, the physiological salt solution contains magnesium sulfate. The concentration of magnesium sulfate in the platelet storage composition can be between about 0.4-0.6 mM, such as about 0.45 mM, 0.5 mM, 0.55 mM, or 0.6 mM, including all ranges and numbers falling within these values. In one embodiment, the concentration of magnesium sulfate is about 0.5 mM. In another embodiment, the platelet storage composition can contain about 0.123 g/L magnesium sulfate. Any form of magnesium sulfate can be used in the present invention, including, without limitation, the heptahydrate form.

### B. Other components

In addition to the physiological salt solution, compositions of the present invention can also include glucose, glutathione, ascorbic acid, arginine, citrulline malate, adenosine, creatine, and less than 1 µM calcium ions (for example, calcium ions supplied by calcium salts such as calcium chloride).

A sugar, for example, a six carbon sugar like glucose (such as D-glucose or dextrose) or a five carbon sugar (such as ribose) can serve as a substrate for the production of high energy phosphates (such as ATP) and can be included in the platelet preservation and storage compositions described herein at concentrations between about 5-15 mM, such as any of about 5 mM, 6 mM, 7 mM, 8 mM, 9 mM, 10 mM, 11 mM, 12 mM, 13 mM, 14 mM, or 15 mM, including all ranges and numbers falling within these values. In one embodiment, the concentration of glucose is about 1.98 g/L. In another embodiment, glucose is present at a concentration of about 11 mM.

Reactive oxygen species can be generated during platelet storage; however, ascorbic acid and reduced glutathione (i.e. reducing agents) present in the solution can consume oxygen free radicals during storage. As such, both ascorbic acid and reduced glutathione can be present in the platelet preservation and storage compositions described herein at concentrations between about 0.5 mM to 3 mM, such as any of about 0.5 mM, 1 mM, 1.5 mM, 2 mM, 2.5 mM, or 3 mM, including all ranges and numbers falling within these values. In one embodiment, the concentration of ascorbic acid is about 0.178 g/L. In another embodiment, ascorbic acid is present at a concentration of about 1 mM. In some embodiments, the concentration of reduced glutathione is about 0.462 g/L. In another embodiment, reduced glutathione is present at a concentration of about 1.5 mM.

Other components of the platelet storage compositions disclosed herein assist in the production of ATP via the tricarboxylic acid (TCA) cycle. In the citrulline malate-arginine cycle, malate (cleaved from citrulline) enters the TCA cycle to generate more ATP. Also, citrulline malate is converted to arginine and fumarate; fumarate enters the TCA cycle to facilitate more ATP production. Both malate and fumarate in TCA cycle leads to more ATP production. Accordingly, arginine (such as L-arginine) and citrulline malate (such as L-citrulline malate) can be present in the platelet preservation and storage compositions described herein at concentrations between about 0.5 mM to 7 mM, such as any of about 0.5 mM, 1 mM, 1.5 mM, 2 mM, 2.5 mM, 3 mM, 3.5 mM, 4 mM, 4.5 mM, 5 mM, 5.5 mM, 6 mM, 6.5 mM, or 7 mM, including all ranges and numbers falling within these values. In one embodiment, the concentration of arginine is about 1.074 g/L. In another embodiment, arginine is present at a concentration of about 5 mM. In some embodiments, the concentration of citrulline malate is about 0.175 g/L. In another embodiment, citrulline malate is present at a concentration of about 0.175 mM. Optionally, citrulline (such as L-citrulline), which facilitates NO synthesis via the arginine succinate and fumerate cycle and also can facilitate ATP synthesis in the Kreb's Cycle via the intermediate fumerate can be added individually to the compositions in ranges of about 1-10 mM citrulline (such as any of about 1 mM, 2 mM, 3 mM, 4 mM, 5 mM, 6 mM, 7 mM, 8 mM, 9 mM, or 10 mM, including all ranges and numbers falling within these values) and about 1-5 mM malic acid (such as any of about 1 mM, 2 mM, 3 mM, 4 mM, or 5 mM, including all ranges and numbers falling within these values), respectively.

Another component useful for maintaining ATP levels is adenosine. Adenosine can be present in the compositions disclosed herein at concentrations between about 1-4 mM, such as any of about 1 mM, 1.5 mM, 2 mM, 2.5 mM, 3 mM, 3.5 mM, or 4 mM, including all ranges and numbers falling within these values. In one embodiment, the concentration of Adenosine is about 0.534 g/L. In another embodiment, Adenosine is present at a concentration of about 2 mM.

In other embodiments, orotic acid is included in the platelet preservation and storage compositions disclosed herein. Orotic acid can be present in concentrations of between about 0.5-2 mM, such as any of about 0.5 mM, 1 mM, 1.5 mM, or 2 mM, including all ranges and numbers falling within these values. In one embodiment, the concentration of Adenosine is about 0.274 g/L. In another embodiment, Adenosine is present at a concentration of about 0.5 mM.

In some embodiments of the platelet storage compositions disclosed herein, the composition solution contains creatine. The concentration of creatine in the platelet storage composition can be between about 2-5 mM, such as about 2 mM, about 3 mM, about 4 mM or about 5 mM. In another embodiment, the platelet storage composition can contain 0.373 g/L potassium creatine. Any form of creatine can be used in the present invention, including, without limitation, creatine monohydrate. However, in some embodiments of any of the compositions described herein, creatine orotate is not included in the composition.

In some embodiments of the compositions disclosed herein, the composition solution contains a buffer for intracellular acidity, such as carnosine (for example, L-carnosine). The concentration of carnosine in the compositions can be between about 5-10 mM, such as about 5 mM, about 6 mM, about 7 mM, about 8 mM, about 9 mM, or about 10 mM, including all ranges and numbers falling within these values. In another embodiment, compositions can contain about 2.26 g/L L-carnosine.

In other embodiments of the compositions disclosed herein, the solution contains carnitine (for example, L-carnitine), which facilitates a decrease in myocardial lactate production, hence reducing acidity. The concentration of carnitine in the compositions can be between about 5-10 mM, such as about 5 mM, about 6 mM, about 7 mM, about 8 mM, about 9 mM, or about 10 mM, including all ranges and numbers falling within these values. In another embodiment, the compositions can contain about 2 g/L L-carnitine.

Dichloroacetate can control acidity by lowering lactate levels in the solution. It can also facilitate lactate entrance into the Krebs cycle via the PDH pathway. The concentration of dichloroacetate in the compositions can be between about 0.5-2.5 mM, such as about 0.5 mM, about 0.6 mM, about 0.7 mM, about 0.8 mM, about 0.9 mM, about 1 mM, about 1.1 mM, about 1.2 mM, about 1.3 mM, about 1.4 mM, about 1.5 mM, about 1.6 mM, about 1.7 mM, about 1.8 mM, about 1.9 mM, about 2 mM, about 2.1 mM, about 2.2 mM, about 2.3 mM, about 2.4 mM, or about 2.5 mM, including all ranges and numbers falling within these values. In another embodiment, the compositions can contain about 0.08 g/L dichloroacetate. In other embodiments, the concentration of dichloroacetate in the compositions can be between about 0.001 mM and 0.5 mM, such as any of about 0.001 mM, about 0.01 mM, about 0.1 mM, about 0.15 mM, about 0.2 mM, about 0.25 mM, about 0.3 mM, about 0.35 mM, about 0.4 mM, about 0.45 mM, or about 0.5 mM. In some embodiments, dichloroacetate is present in compositions for storing platelets at ambient temperatures (for example, between about 21 ± 2°C). In other embodiments, dichloroacetate is not present in compositions for storing platelets at sub-ambient temperatures (for example, from about 0 °C to about 18 °C).

In some embodiments of the platelet storage compositions disclosed herein, the compositions do not contain one or more of calcium ions, creatine orotate, dichloroacetate, citrulline (for example, citrulline malate), or insulin.

The platelet storage compositions disclosed herein are maintained at a neutral or slightly basic pH, namely, about pH 7, 7.1, 7.2, 7.3, 7.4, 7.5, 7.6, or 7.7, including all ranges and numbers falling within these values. In one embodiment, the pH of the platelet storage composition is 7.5.

In other embodiments, the platelet storage composition comprises the following base or nominal ingredients as shown in Table I combined in deionized and/or bacteriostatic water:

**Table I: Exemplary formulation for platelet storage solution**

| Component | Amount |
|---|---|
| Distilled water | 1L |
| Calcium ions | < 1µM |
| Potassium chloride | 4-5 mM |
| Potassium phosphate | 0.44-10 mM |
| Magnesium chloride | 0.5-2.5 mM |
| Magnesium sulfate | 0.5-2.5 mM |
| Sodium chloride | 100-115 mM |
| Sodium bicarbonate | 5-15 mM |
| Sodium phosphate | 10-40 mM |
| Dichloroacetate | 0-2.5 mM |
| Glutathione | 1.5-5.0 mM |
| Ascorbic acid | 1.0-5 mM |
| Adenosine | 2-5 mM |

In some embodiments, the potassium phosphate salt for use in the non-limiting formulation shown in Table I can be potassium phosphate monobasic. In another embodiment, the magnesium chloride salt for use in the non-limiting formulation shown in Table I can be magnesium chloride hexahydrate. In other embodiments, the magnesium sulfate salt for use in the non-limiting formulation shown in Table I can be magnesium sulfate heptahydrate. In yet other embodiments, the sodium phosphate salt for use in the non-limiting formulation shown in Table I can be sodium phosphate dibasic heptahydrate. In some embodiments, the glutathione for use in the non-limiting formulation shown in Table I can be reduced glutathione. In another embodiment, the creatine for use in the non-limiting formulation shown in Table I can be creatine monohydrate.

In further embodiments, the non-limiting formulation shown in Table I can further comprise one or more of arginine (for example, L-arginine) in concentrations of between about 2 to about 10 mM, such as any of about 2 mM, 3 mM, 4 mM, 5 mM, 6 mM, 7 mM, 8 mM, 9 mM or 10 mM, carnosine (for example, L-carnosine) in concentrations of between about 5 to about 10 mM, such as any of about 5 mM, 6 mM, 7 mM, 8 mM, 9 mM, or 10 mM (2.26 g/L for 10 mM), carnitine (for example, L-carnitine) in concentrations of between about 5 to about 10 mM, such as any of about 5 mM, 6 mM, 7 mM, 8 mM, 9 mM, or 10 mM (2.26 g/L for 10 mM), orotic acid, for example, in concentrations of about 0.5-2 mM, such as any of about 0.5, 1, 1.5, or 2 mM, or creatine (for example, creatine monohydrate), in concentrations of about 2-5 mM, such as any of about 2 mM, 3 mM, 4 mM, or 5 mM.

In yet other embodiments, the non-limiting formulation shown in Table I can further comprise a sugar, such as, but not limited to, a six carbon sugar (e.g., allose, altrose, galactose, glucose (including D-glucose (a.k.a. dextrose) and L-glucose), gulose, idose, mannose, talose, fructose, psicose, sorbose, tagatose, fucose, fuculose, or rhamnose) or a five carbon sugar (e.g. arabinose, lyxose, ribose, xylose, ketopentoses, ribulose, or xylulose) in concentrations from between about 11 mM to about 25 mM, such as any of about 11 mM, 12 mM, 13 mM, 14 mM, 15 mM, 16 mM, 17 mM, 18 mM, 19 mM, 20 mM, 21 mM, 22 mM, 23 mM, 24 mM, or 25 mM sugar.

In still other embodiments, the non-limiting formulation shown in Table I can optionally comprise 1-10 mM of citrulline (for example, L-citrulline) or a salt thereof in concentrations of between about 2 to about 10 mM, such as any of about 2 mM, 3 mM, 4 mM, 5 mM, 6 mM, 7 mM, 8 mM, 9 mM, or 10 mM. In another embodiment, the non-limiting formulation shown in Table II can optionally comprise about 0-10 mM malic acid, such as any of about 0 mM, 1 mM, 2 mM, 3 mM, 4 mM, 5 mM, 6 mM, 7 mM, 8 mM, 9 mM, or 10 mM. In another embodiment, the non-limiting formulation shown in Table II can optionally comprise citrulline malate (such as L-citrulline malate) instead of malic acid and/or citrulline in concentrations of about 0 mM to about 10 mM or about 2 mM to about 7 mM, such as any of about 0 mM, 1 mM, 2 mM, 3 mM, 4 mM, 5 mM, 6 mM, 7 mM, 8 mM, 9 mM or 10 mM citrulline malate.

In other embodiments, the non-limiting formulation shown in Table I can optionally include a calcium chelator (such as, but not limited to, one or more of ethylenediaminetetraacetic acid (EDTA), ethyleneglycoltetraacetic acid (EGTA), diethylenetriaminepentaacetate (DTPA), hydroxyethylethylenediaminetriacetic acid (HEEDTA), diaminocyclohexanetetraacetic acid (CDTA), 1,2-bis(2-aminophenoxy)ethane-N,N,N',N'-tetraacetic acid (BAPTA), and citric acid) in concentrations of about 0 mM to about 5mM, such as any of about 0 mM, 1 mM, 2 mM, 3 mM, 4 mM, or 5 mM. Use of a calcium chelator in any of the platelet storage compositions described herein may be necessary when the water for use in combining the various components is not completely or substantially free of calcium ions (*i.e.* the water has a calcium ion concentration of > 100 µM (or > any of about 0 µM , 1 µM, 2 µM, 3 µM, 4 µM, 5 µM, 10 µM, 15 µM, 20 µM, 25 µM, 30 µM, 35 µM, 40 µM, 45 µM, 50 µM, 55 µM, 60 µM, 65 µM, 70 µM, 75 µM, 80 µM, 85 µM, 90 µM, or 95 µM). Detection and quantification of calcium ions in a solution is readily ascertainable by methods known in the art, such as by, for example, atomic absorption spectroscopy.

In other embodiments, and as a non-limiting example, the platelet storage composition comprises the following ingredients as shown in Table II combined in deionized and/or bacteriostatic water:

**Table II: Exemplary formulation for platelet storage solution**

| **Component** | **Amount** |
|---|---|
| Distilled water | 1L |
| Calcium ions | < 1µM |
| Potassium chloride | 4-5 mM |
| Potassium phosphate (monobasic) | 0.44-10 mM |
| Magnesium chloride (hexahydrate) | 0.5-2.5 mM |
| Magnesium sulfate (heptahydrate) | 0.5-2.5 mM |
| Sodium chloride | 100-115 mM |
| Sodium bicarbonate | 5-15 mM |
| Sodium phosphate (dibasic; heptahydrate) | 10-40 mM |
| D-Glucose | 11-25 mM |
| Dichloroacetate | 0-0.5 mM |
| Glutathione (reduced) | 1.5-5.0 mM |
| Ascorbic acid | 1.0-5 mM |
| L-Arginine | 2-10 mM |
| L-Citrulline malate* | 2-10 mM |
| Adenosine | 2-5 mM |
| Orotic acid | 0.5-2 mM (optional) |
| Creatine monohydrate | 2-5 mM (optional) |
| L-Carnosine | 5-10 mM (2.26 gm/L for 10 mM) (optional) |
| L-Carnitine | 5-10 mM (2.00 gm/L for 10 mM) (optional) |
| EGTA | 0-5 mM (optional) |

| | |
|---|---|
| *optionally, 1-10 mM of L-citrulline and 1-5 mM of malic acid may be used instead of L-citrulline malate | |

### III. Method

### A. Methods for preserving platelets

Effective methods for storing platelets using the compositions disclosed herein are also provided by the present invention. Platelets can be stored in the solutions disclosed herein at ambient or room temperature (21 ± 2°C) in platelet transfer bags. The compositions for storing platelets at ambient or room temperatures contain dichloroacetate. In another embodiment, platelets can be stored in the solutions disclosed herein at sub-ambient temperatures (4 ± 1°C or 13 ± 3°C) In yet other embodiments, can be stored in the solutions disclosed herein at temperatures ranging from about 3 °C to about 23 °C, such as any of about 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, or 25 °C.

In some embodiments, platelets stored in the presently disclosed compositions can be gently agitated during all or part of the course of storage, such as on a flatbed shaker or any similar agitation device. According to the methods provided herein, platelets may be stored in the disclosed solutions herein for up to 15 days, such as any of 1, 2, 3, 4, 5, 6, 7, 8, 9, 10, 11, 12, 13, 14, or 15 days, without significant accumulation of storage lesions, *e.g.,* the platelets are suitable for transfusion after > 5 days of storage.

Nitric oxide (NO) is a bioregulatory molecule with diverse functional roles in cardiovascular homeostasis, neurotransmission and immune response. As discussed in the Examples section, the inventors of the present application have discovered that NO production in stored platelets is critical for healthy platelet physiology and is associated with reductions in platelet storage lesions as well as inhibition of platelet aggregation. Accordingly, the ideal storage solution would ensure that NO production in platelets remained normal during the course of storage. Platelets stored in any of the solutions disclosed herein according to the methods disclosed herein exhibit significantly more NO production (such as any of about 10%, 15%, 20%, 25%, 30%, 35%, 40%, 45%, 50%, 55%, 60%, 65%, 70%, or 75%, greater NO production, including all ranges and numbers falling within these percentages) compared to platelets that are not stored in the solutions disclosed herein. In the compositions described herein, components such as citrulline (e.g., citrulline malate) and arginine (e.g., L-arginine) help to maintain NO production in stored platelets.

Maintenance of high energy phosphate concentrations (such as, ATP) in platelets during extended storage is also important for the reduction of storage lesions. Platelets stored in any of the solutions disclosed herein according to the methods disclosed herein exhibit significantly more high energy phosphates (such as any of about 10%, 15%, 20%, 25%, 30%, 35%, 40%, 45%, 50%, 55%, 60%, 65%, 70%, or 75% more high energy phosphates including all ranges and numbers falling within these percentages) compared to platelets that are not stored in the solutions disclosed herein.

Platelets will often aggregate as early as 3 days after being placed in storage. Aggregation of platelets is associated with the onset of storage lesions, so an ideal platelet storage solution would maintain the platelets in a substantially non-aggregated state. Platelets stored in any of the solutions disclosed herein according to the methods disclosed herein exhibit significantly less aggregation (such as any of about 10%, 15%, 20%, 25%, 30%, 35%, 40%, 45%, 50%, 55%, 60%, 65%, 70%, or 75%, less aggregation, including all ranges and numbers falling within these percentages) compared to platelets that are not stored in the solutions disclosed herein. Similarly, platelets stored in the presently disclosed solutions exhibit significantly less alterations in morphology (such as, loss of discoid morphology) compared to platelets that are not stored in the solutions disclosed herein over comparable periods of time.

Another characteristic lesion often observed in stored platelets is the translocation of the phospholipid phosphatidylserine (PS) from the inner to the outer platelet membrane. Platelets stored in any of the solutions disclosed herein according to the methods disclosed herein exhibit significantly less PS translocation (such as any of about 10%, 15%, 20%, 25%, 30%, 35%, 40%, 45%, 50%, 55%, 60%, 65%, 70%, or 75%, less PS translocation, including all ranges and numbers falling within these percentages) compared to platelets that are not stored in the solutions disclosed herein.

Platelets stored for prolonged periods of time exhibit significant increases in lactate production, which can negatively affect the pH of the storage media leading to increased storage-mediated lesions. Platelets stored in any of the solutions disclosed herein according to the methods disclosed herein exhibit significantly less lactate production (such as any of about 10%, 15%, 20%, 25%, 30%, 35%, 40%, 45%, 50%, 55%, 60%, 65%, 70%, or 75%, less lactate production, including all ranges and numbers falling within these percentages) compared to platelets that are not stored in the solutions disclosed herein.

### B. Methods for producing platelet storage compositions

Provided herein are methods for producing a composition for preserving platelets, such as any of the compositions disclosed herein. The methods encompass mixing one or more of the following ingredients at the indicated concentrations in Table III in distilled, deionized, and/or bacteriostatic water to produce a non-limiting example of a platelet storage composition.

**Table III:**

| **Component** | **mM** |
|---|---|
| Calcium ions | < 1µM |
| Potassium Chloride | 4.0 |
| Potassium phosphate | 0.44 |
| Magnesium chloride | 0.5 |
| Magnesium sulfate | 0.5 |
| Sodium chloride | 100.00 |
| Sodium bicarbonate | 5.00 |
| Sodium phosphate | 30.00 |
| Glucose or dextrose | 11.00 |
| Dichloroacetate | 0.50 |
| Glutathione | 1.50 |
| Ascorbic acid | 1.00 |
| Arginine | 5.00 |
| Malic acid | 1.00 |
| Adenosine | 2.00 |
| Orotic acid | 0.50 |
| Creatine | 2.50 |
| Carnosine | 10 |
| Carnitine | 10 |
| EGTA | 2.5 (Optional) |

In one embodiment of the methods disclosed herein, rather than adding citrulline malate (such as, L-citrulline malate) to the composition, 1-10 mM (such as any of about 1 mM, 2 mM, 3 mM, 4 mM, 5 mM, 6 mM, 7 mM, 8 mM, 9 mM, or 10 mM, including all ranges and numbers falling within these values) of citrulline (such as, L-citrulline) can be added along with 1-5 mM (such as any of about 1 mM, 2 mM, 3 mM, 4 mM, or 5 mM, including all ranges and numbers falling within these values) of malic acid, respectively.

In some embodiments, the potassium phosphate salt for use in producing the non-limiting formulation shown in Table III can be potassium phosphate monobasic. In another embodiment, the magnesium chloride salt for use in the non-limiting formulation shown in Table III can be magnesium chloride hexahydrate. In other embodiments, the magnesium sulfate salt for use in the non-limiting formulation shown in Table III can be magnesium sulfate heptahydrate. In yet other embodiments, the sodium phosphate salt for use in the non-limiting formulation shown in Table III can be sodium phosphate dibasic heptahydrate. In some embodiments, the glutathione for use in the non-limiting formulation shown in Table III can be reduced glutathione. In another embodiment, the creatine for use in the non-limiting formulation shown in Table III can be creatine monohydrate. In another embodiment, the arginine for use in the non-limiting formulation shown in Table III can be L-arginine. In another embodiment, the carnosine for use in the non-limiting formulation shown in Table III can be L-carnosine. In another embodiment, the carnitine for use in the non-limiting formulation shown in Table III can be L- carnitine.

In yet other embodiments, methods for producing a composition for preserving platelets encompass mixing one or more of the following ingredients at the indicated concentrations in Table IV in distilled, deionized, and/or bacteriostatic water to produce a non-limiting example of a platelet storage composition.

**Table IV:**

| **Component** | **mM** |
|---|---|
| Calcium ions | < 1µM |
| Potassium Chloride | 4.0 |
| Potassium phosphate | 0.44 |
| Magnesium chloride | 0.5 |
| Magnesium sulfate | 0.5 |
| Sodium chloride | 100.00 |
| Sodium bicarbonate | 5.00 |
| Sodium phosphate | 30.00 |
| Dichloroacetate | 0.50 ( |
| Glutathione | 1.50 |
| Ascorbic acid | 1.00 |
| Adenosine | 2.00 |
| Calcium chelator | 2.5 (Optional) |

In some embodiments, the potassium phosphate salt for use in the non-limiting formulation shown in Table IV can be potassium phosphate monobasic. In another embodiment, the magnesium chloride salt for use in the non-limiting formulation shown in Table IV can be magnesium chloride hexahydrate. In other embodiments, the magnesium sulfate salt for use in the non-limiting formulation shown in Table IV can be magnesium sulfate heptahydrate. In yet other embodiments, the sodium phosphate salt for use in the non-limiting formulation shown in Table IV can be sodium phosphate dibasic heptahydrate. In some embodiments, the glutathione for use in the non-limiting formulation shown in Table IV can be reduced glutathione.

In further embodiments, the non-limiting formulation shown in Table IV can further comprise one or more of arginine (for example, L-arginine) in concentrations of between about 2 to about 10 mM, such as any of about 2 mM, 3 mM, 4 mM, 5 mM, 6 mM, 7 mM, 8 mM, 9 mM or 10 mM, carnosine (for example, L-carnosine) in concentrations of between about 5 to about 10 mM, such as any of about 5 mM, 6 mM, 7 mM, 8 mM, 9 mM, or 10 mM (2.26 g/L for 10 mM), carnitine (for example, L-carnitine) in concentrations of between about 5 to about 10 mM, such as any of about 5 mM, 6 mM, 7 mM, 8 mM, 9 mM, or 10 mM (2.26 g/L for 10 mM), orotic acid, for example, in concentrations of about 0.5-2 mM, such as any of about 0.5, 1, 1.5, or 2 mM, or creatine (for example, creatine monohydrate), in concentrations of about 2-5 mM, such as any of about 2 mM, 3 mM, 4 mM, or 5 mM.

In yet other embodiments, the non-limiting formulation shown in Table IV can further comprise a sugar, such as, but not limited to, a six carbon sugar (e.g., allose, altrose, galactose, glucose (including D-glucose (a.k.a. dextrose) and L-glucose), gulose, idose, mannose, talose, fructose, psicose, sorbose, tagatose, fucose, fuculose, or rhamnose) or a five carbon sugar (e.g. arabinose, lyxose, ribose, xylose, ketopentoses, ribulose, or xylulose) in concentrations from between about 11 mM to about 25 mM, such as any of about 11 mM, 12 mM, 13 mM, 14 mM, 15 mM, 16 mM, 17 mM, 18 mM, 19 mM, 20 mM, 21 mM, 22 mM, 23 mM, 24 mM, or 25 mM of sugar.

In still other embodiments, the non-limiting formulation shown in Table IV can optionally comprise 1-10 mM of citrulline (for example, L-citrulline) or a salt thereof in concentrations of between about 2 to about 10 mM, such as any of about 2 mM, 3 mM, 4 mM, 5 mM, 6 mM, 7 mM, 8 mM, 9 mM, or 10 mM. In another embodiment, the non-limiting formulation shown in Table IV can optionally comprise about 0-10 mM malic acid, such as any of about 0 mM, 1 mM, 2 mM, 3 mM, 4 mM, 5 mM, 6 mM, 7 mM, 8 mM, 9 mM, or 10 mM. In another embodiment, the non-limiting formulation shown in Table IV can optionally comprise citrulline malate (such as L-citrulline malate) instead of malic acid and/or citrulline in concentrations of about 0 mM to about 10 mM or about 2 mM to about 7 mM, such as any of about 0 mM, 1 mM, 2 mM, 3 mM, 4 mM, 5 mM, 6 mM, 7 mM, 8 mM, 9 mM or 10 mM citrulline malate.

In other embodiments, the non-limiting formulation shown in Table IV can optionally include a calcium chelator (such as, but not limited to, one or more of ethylenediaminetetraacetic acid (EDTA), ethyleneglycoltetraacetic acid (EGTA), diethylenetriaminepentaacetate (DTPA), hydroxyethylethylenediaminetriacetic acid (HEEDTA), diaminocyclohexanetetraacetic acid (CDTA), 1,2-bis(2-aminophenoxy)ethane-N,N,N',N'-tetraacetic acid (BAPTA), and citric acid) in concentrations of about 0 mM to about 5mM, such as any of about 0 mM, 1 mM, 2 mM, 3 mM, 4 mM, or 5 mM. Use of a calcium chelator for producing any of the platelet storage compositions described herein is necessary when the water is not completely or substantially free of calcium ions (i.e. the water has a calcium ion concentration of > 0 µM or > 1 µM). Detection and quantification of calcium ions in a solution is readily ascertainable by methods known in the art, such as by, for example, atomic absorption spectroscopy.

The method includes a step of adjusting the pH of the solution to a neutral or slightly basic level, such as about pH 7, 7.1, 7.2, 7.3, 7.4, 7.5, 7.6, or 7.7, including all ranges and numbers falling within these values. In one embodiment, the pH of the platelet storage composition is adjusted to 7.5.

In yet other embodiments, methods for producing a composition for preserving platelets encompass mixing one or more of the following ingredients at the indicated concentrations in Table V in distilled, deionized, and/or bacteriostatic water to produce a non-limiting example of a platelet storage composition.

**Table V: Exemplary formulation for platelet storage solution**

| **Component** | **Amount** |
|---|---|
| Distilled water | 1L |
| Calcium ions | < 1µM |
| Potassium chloride | 4-5 mM |
| Potassium phosphate | 0.44-10 mM |
| Magnesium chloride | 0.5-2.5 mM |
| Magnesium sulfate | 0.5-2.5 mM |
| Sodium chloride | 100-115 mM |
| Sodium bicarbonate | 5-15 mM |
| Sodium phosphate | 10-40 mM |
| D-Glucose | 11-25 mM |
| Dichloroacetate | 0-0.5 mM ( |
| Glutathione | 1.5-5.0 mM |
| Ascorbic acid | 1.0-5 mM |
| Arginine | 2-10 mM |
| Citrulline | 1-10 mM |
| Malic acid | 0-7 mM (optional) |
| Adenosine | 2-5 mM |
| Orotic acid | 0.5-2 mM |
| Creatine | 2-5 mM |
| Carnosine | 5-10 mM (2.26 gm/L for 10 mM) |
| Carnitine | 5-10 mM (2.00 gm/L for 10 mM) |
| EGTA | 0-5 mM (optional) |

### C. Methods for detecting the presence of nitric oxide in platelets in real time

Also provided herein are methods for detecting the presence of basal nitric oxide (NO) in platelets in real time. These methods provide a relatively rapid and accurate technique that can be used in conjunction with the disclosed platelet storage compositions and related methods to monitor the health of platelets throughout the course of the storage period.

The methods encompass labeling a sample containing stored platelets with a nitric oxide-specific fluorescent dye. Any nitric oxide-specific fluorescent dye can be used in conjunction with the methods and include those that are commercially available (such as, for example, DAF-FM, 2,3-Diaminonaphthalene, 1,2-Diaminoanthraquinone, or NBD Methylhydrazine, available from Life Technologies or DAF-2 DA available from Enzo Life Sciences).

Following labeling with a nitric oxide-specific fluorescent dye, the platelets can be optionally fixed using any appropriate fixative known in the art, such as, but not limited to gluteraldehyde, formalin, formaldehyde, ethanol, methanol, osmium tetroxide, potassium dichromate, chromic acid, or potassium permanganate. Fixation can be performed, for example, on a microscope slide or any other medium suitable for fluorescence imaging.

Fluorescently labeled platelets can be assessed for NO production using any means known in the art for detecting fluorescent light, such as, but not limited to, fluorescent microscopy (such as confocal microscopy), flow cytometric techniques such as Fluorescence-activated cell sorting (FACS), or fluorometers.

### IV. Kits

The compositions for making the storage/resuscitation solution are optionally packaged in a kit with the ingredients listed below or multiples thereof in amounts necessary to scale up to make 2, 3, 5, 10, 20 times the amount of solution. An exemplary kit contains one or more of glutathione, ascorbic acid, adenosine, potassium chloride, potassium phosphate magnesium chloride, magnesium sulfate, sodium chloride, sodium bicarbonate, sodium phosphate, a sugar (such as ribose, glucose or dextrose), arginine, citrulline malate, adenosine, orotic acid, creatine, a calcium chelator (*e.g.,* EGTA) and dichloroacetate (for example, one or more of about 0.3 g/L Potassium Chloride, 0.06 g/L Potassium phosphate (monobasic), 0.110 g/L Magnesium chloride (hexahydrate), 0.123 g/L Magnesium sulfate (heptahydrate), 5.84 g/L Sodium chloride, 0.42 g/L Sodium bicarbonate, 7.9 g/L Sodium phosphate (dibasic; heptahydrate), 1.98 g/L D-Glucose, 0.462 g/L Glutathione (reduced), 0.178 g/L Ascorbic acid, 1.074 g/L Arginine, 0.175 g/L L-Citrulline malate, 0.534 g/L Adenosine, 0.274 g/L Orotic acid, 0.373 g/L, Creatine monohydrate, 0.001-5 mM EGTA, and/or 0.075 gm/L dichloroacetate). The kit may optionally also contain citrulline (such as L-citrulline), and/or malic acid.

These ingredients can be packaged together with instructions for use and are mixed in 0.01-2.0 L of distilled water. The kit may also contain solutions of means for adjusting the pH of the combined platelet preservation solution (e.g. THAM). The kit can be packaged or sold with or without the sterile water component.

Also provided herein are kits for detecting the presence of nitric oxide in platelets in real time. These kits can encompass one or more nitric oxide-specific fluorescent dyes and/or means for measuring a fluorescent signal, such as, but not limited to, a fluorometer. Other components of the kits can include a fixation reagent, microscope slides, and written instructions for performing the platelet nitric oxide assessment assay.

The invention can be further understood by reference to the following examples, which are provided by way of illustration and are not meant to be limiting.

### EXAMPLES

### Example 1

In the following Examples, tables are designated using Arabic numbers (e.g. Table 1, Table 2, Table 3, etc.). This Example shows that platelets stored in Aayusol solution exhibited increased production of nitric oxide as well as decreased levels of aggregation in comparison to platelets stored in PASIII-M.

### Materials and Methods

Platelet concentrates and platelet rich plasma preparation and storage: PASIII-M (Kaufman Hematology Am Soc Hematol Educ Program. 2006, 492-496; Alhumaidan J Clinical Apheresis 2012; 27:93-98) and Aayusol (pH 7.5) were prepared, the composition of which is shown in Table 1. Platelet concentrates were obtained from Research Blood Components (Cambridge, MA). Platelet rich plasma (PRP) from 3 consented healthy volunteer donors was prepared as described (Hou et al. 2 Vox Sanguinis (2011) 100, 187-195; Gao et al. Thromb Haemost 2012; 107: 681-689). Briefly, venous blood (40-60 ml) was collected from healthy volunteers into tubes containing standard anticoagulant ACD-A (9:1, vol/vol). Samples were centrifuged (15 min at 200g), and top two-thirds of platelet-rich plasma (PRP) was collected. Harvested platelets (PRP) were transferred into gas-permeable storage bags containing PASIII-M or Aayusol (20:80 v/v). All samples were significantly leuko-reduced with white blood cells counts less than 5 x 10⁶/unit. Sample units were standardized to contain more than 3 x 10¹¹ platelets in accordance with American Association of Blood Banks standards.

**Table 1: Composition of Aayusol solution**

| **Component** | **mM** | **g/L** |
|---|---|---|
| Potassium Chloride | 4.00 | 0.300 |
| Potassium phosphate (monobasic) | 0.44 | 0.060 |
| Magnesium chloride (hexahydrate) | 0.50 | 0.111 |
| Magnesium sulfate (heptahydrate) | 0.50 | 0.123 |
| Sodium chloride | 100.00 | 5.840 |
| Sodium bicarbonate | 5.00 | 0.420 |
| Sodium phosphate (dibasic; heptahydrate) | 30.00 | 7.900 |
| D-Glucose | 11.00 | 1.980 |
| Dichloroacetate | 0.50 | 0.075 gm/L |
| Glutathione (reduced) | 1.50 | 0.462 |
| Ascorbic acid | 1.00 | 0.178 |
| L-Arginine | 5.00 | 1.074 |
| L-Citrulline malate | 1.00 | 0.175 |
| Adenosine | 2.00 | 0.534 |
| Orotic acid | 0.50 | 0.274 |
| Creatine monohydrate | 2.50 | 0.373 |
| carnosine | 10.00 | 2.26 |
| carnitine | 10.00 | 2.0 |

Platelet transfer bags were stored at 21±2°C with constant gentle agitation on a flatbed shaker (Helmer PC 100, Noblesville, IN) under sterile conditions. Stored platelets were sampled gently for temporal evaluation during 9-14 day storage. Platelets were stored in autologous blood transfer bags (300 ml capacity; Medtronics, Minneapolis, MN; cat No. ATBAG300).

Confocal microscopy: Platelets were labeled periodically with DAF2-DA (50 µM, a nitric oxide specific fluorescence dye) by incubation for 60 min at room temperature. Platelets were rapidly fixed in 1% glutaraldehyde and imaged using confocal microscopy. Samples were excited with a 488 nm krypton-argon laser, and narrow band pass filters were used to restrict emission wavelength overlap. The platelets were scanned and imaged on a confocal microscope (Carl Zeiss GmbH, Jena, Germany).

Flow-cytometric analysis of nitric oxide synthesis: Platelets stored from 1-9 days in PASIII-M (Kaufman Hematology Am Soc Hematol Educ Program. 2006, 492-496; Alhumaidan J Clinical Apheresis 2012; 27:93-98) and Aayusol were labeled periodically with DAF2-DA by incubation for 60 min at room temperature. Then, the mixture was diluted in 200 µl of Tyrode's buffer or Aayusol solution and assessed by flow cytometry. Events with less than 1 to 6 µm diameter were identified in forward scatter and side scatter intensity dot representations. Acquisition was performed for 1 min per sample, during which flow cytometry analyzed approximately 60 µl of the suspension. Microparticle concentration was calculated as previously described (Hou; Gao). Results were expressed as number of microparticle per ml. Every sample was repeated at least three times and the mean value was obtained. The samples were analyzed using BD LSR Fortessa (Becton-Dickinson, San Jose, CA).

### Results

Platelets were preserved in PASIII-M and Aayusol (20:80) in standard 300 ml transfer bags (Medtronics) for 9 days at ambient temperature with gentle shaking. Figure 1 shows a representative image of n=5 experiments; 200X magnification. Images show that after day 5 of storage, platelets in Aayusol were producing greater amount of tonic nitric oxide as indicated by brightness of green fluorescence as compared to those in PAS. Additionally, platelets maintained their morphology and did not demonstrate any aggregation in Aayusol. In contrast, platelets appeared to be swollen (partially activated) and started forming aggregates (*see* Figure 1A (left inset)) in PASIII-M during storage, as early as 3 days. In contrast, partial aggregation of platelets was observed in Aayusol only after 9-day storage. This is the first demonstration of the measurement of nitric oxide generation in platelets using a fluorescence imaging technique. As shown in Figure 1B, DAF-labeled samples were analyzed using FACS scanner. Total fluorescence (photon) counts are expressed in arbitrary units. Nitric oxide generation decreased with time but was significantly greater in platelets preserved in Aayusol over the storage period. There was almost an instantaneous decrease in basal production of nitric oxide in platelets stored in PASIII-M. In contrast, basal nitric oxide generation was robustly maintained in platelets preserved in Aayusol throughout the 9-day storage period. These results demonstrate that in contrast to PASIII-M, the minimally observed aggregation of platelets in Aayusol is the result of robust production of nitric oxide by the stored platelets, as nitric oxide decreases platelet aggregation and activation. (Data represents mean ± SD, n=5, p<0.005).

### Example 2

This Example shows that preservation of high-energy phosphates facilitates the maintenance of homeostasis in stored platelets.

### Materials and Methods

ATP and Creatine Phosphate Assay: ATP and creatine phosphate (CP) were measured in platelet extracts as described (Besho et al. 1991). In brief, platelets were suspended in 400 µl of 0.4M ice-cold perchloric acid and homogenized twice for 30-sec. Homogenate was centrifuged at 1970g for 10-mins at 0°C. An aliquot of supernatant was neutralized with equal volume of ice-cold 0.4M KHCO₃ solution and centrifuged as described above. The supernatant was stored at -80°C for ATP (and CP) measurements. The pellet was dissolved in equal volume of 0.1M NaOH and centrifuged and used for protein assay. ATP and CP were measured using a bioluminescent assay kit (Sigma-Aldrich and GloMax-Multi+ Detection System, Promega) according to the protocol provided by manufacturer.

### Results

Results are shown in Figure 2. Platelets were stored in Aayusol and current standard PASIII-M for 15 days at ambient temperature (21± 2°C) with gentle shaking. PRP (Platelet rich plasma) was used as a control. Platelets were periodically evaluated for ATP and creatine phosphate (CP) concentration. There was a temporal decrease in ATP and CP concentration (pMoles) during storage. However, ATP and CP concentration in platelets stored in Aayusol was significantly greater than in PP and platelets stored in PASIII-M after 9-days of storage. (Data represents mean ± SD; n=3, p<0.05).

### Example 3

This Example demonstrates that preservation of platelets in Aayusol attenuates phosphatidylserine (PS) exposure and induction of apoptosis during long-term storage.

### Materials and Methods

Phosphatidylserine (PS) Exposure: Activated platelets, their aggregates, and microparticles were detected by their ability to bind FITC-lactadherin (Haematologic Technologies, Inc., Essex Junction, VT, USA), which interacts with phosphatidylserine (PS) exposed during activation and early apoptosis, as reported previously (Hou et al. 2 Vox Sanguinis (2011) 100, 187-195; Gao et al. Thromb Haemost 2012; 107: 681-689).

Flow-cytometric analysis of PS exposure: Platelets were stored at ambient temperature of 21±2°C in Aayusol for 0, 1, 2, 3, 4, 5, 7, 8, and 9 days. Platelets (50µl) were resuspended in Tyrode's buffer or Aayusol solution and were incubated with 5 µl of FITC-lactadherin for 10 min at room temperature in the dark. Then, the mixture was diluted in 200 µl of Tyrode's buffer or Aayusol solution and assessed by flow cytometry. Events with less than 1 to 6 µm diameter were identified in forward scatter and side scatter intensity dot representation. Acquisition was performed for 1 min per sample, during which flow cytometry analyzed approximately 60 µl of the suspension. PS exposure on platelets, microparticles, and aggregates were positively labelled by lactadherin/anti-CD41. Microparticle concentration was calculated as previously described (Hou et al. 2 Vox Sanguinis (2011) 100, 187-195; Gao et. al. Thromb Haemost 2012; 107: 681-689). Results were expressed as number of microparticle per ml. Every sample was repeated at least three times and the mean value was obtained. The samples were analyzed using BD LSR Fortessa (Becton-Dickinson, San Jose, CA).

Confocal microscopy: A coverslip was coated with 10 µg/ml fibrinogen in PBS for 2 h at room temperature, washed 3X with Tyrode's buffer, blocked with BSA (bovine serum albumin) for 30 min and then washed twice with Tyrode's buffer. Two hundred microliters of stored platelet suspension was transferred to the slides and incubated for 40 min at room temperature (RT). After the incubation, slides were washed and incubated with 5 µl of FITC-lactadherin for 10 min in the dark at RT. Thereafter, the incubated mixture was washed in 1 ml of Aayusol to remove the unbound dye. Samples were excited with a 488 nm krypton-argon laser, and narrow band pass filters were used to restrict emission wavelength overlap. The platelets were scanned and imaged on a confocal microscope (Carl Zeiss GmbH, Jena, Germany). Cells were imaged using Zeiss LSM 710 confocal microscope using 63X oil immersion objective.

Blood chemistry: Dissolved O₂, CO₂, bicarbonate, base efficiency, ionic composition and lactate levels in the stored platelet media were periodically estimated using Abaxis Vetscan VS2 or i-Stat System using CG4 and CG8 cartridges.

### Results

Figure 3A shows flow-cytometric analysis of PS exposure in stored platelets. Samples were analyzed using 488 nm excitation and 530 nm emission wavelengths, respectively. The results showed that only 7.7 +/- 0.5 % of platelets stored in Aayusol (Fig. 3A top row) show PS exposure at the end of 9-day storage. In contrast, and 29.20 +/- 3 % of platelets stored in PAS-IIIM (Fig. 3A bottom row) were significantly PS positive (dark blue arrows) indicating potential induction of apoptosis. Platelet preservation in Aayusol was significantly superior (p<0.001; n=5) to those in PASIIIM. Figure 3B shows flow-cytometric analysis of aggregate (light blue arrows) and micro particle (black arrows) formation in stored platelets. In Fig 3B, top panel, platelets stored in Aayusol demonstrate minimal aggregation and micro particle formation during 9-day storage. In contrast, extensive aggregation and micro particle formation was demonstrated by platelets stored in PASIII-M solution. These results clearly indicate superiority of platelet storage in Aayusol compared to currently used PASIII-M solution.

Temporal measurement of lactate production is shown in Table 2.

**Table 2: Lactate production (mMoles/Liter) in stored platelets**

| **Storage Days** | **0** | **3** | **5** | **7** | **9** | |
|---|---|---|---|---|---|---|
| PASIII-M | 0.72±0.35 | | 3.85±0.91 | | 6.50±1.40 | n = 3 |
| Aayusol | 0.85±0.26 | 0.99±0.30 | 1.76±0.63 | 2.63±0.90 | 3.47±1.26 | n = 3 |

Figure 4 demonstrates the expression of P-Selectin on platelets during storage. Platelets were stored in Aayusol and PASIII-M for 9 days as described. Platelets were temporally labeled with FITC-anti CD62 antibody and assayed using FACS. P-Selection expression, a marker of activation, was substantially attenuated on platelets stored in Aayusol. In contrast, there was a significant increase in P-Selectin expression on platelets stored in PASIII-M during the entire 9-day storage. These results demonstrate that platelets are activated during storage in PASIII-M, but minimally so in Aayusol demonstrating superiority of preservation. (Data represents mean ± SD; n=3, p<0.001).

Figure 5 is an image of confocal microscopy of stored platelets. Platelets were stored in PASIII-M or Aayusol for 0-9 days and were periodically labeled with FITC-lactadherin to identify PS exposure (induction of apoptosis). Platelets showed progressive increase in PS exposure. On day 0 and 1, discoid-type platelets with or without pseudopods showed no lactadherin staining. By day 3-7, the morphology of the platelets changed into irregular shapes, and PS exposure was found on the scattered and ruffled regions of platelets and microparticles. By day 7-9, more platelets had lactadherin-positive membranes, which were characterized by cell shrinkage, pseudopods at cellular rim areas and occasional rounded protuberances. By day 9, the intensity of PS exposure had increased while the cytoplasm had condensed, the membrane had ruffled, more vesiculation/blebs had formed and large amounts of long filopodia-like extensions had appeared and cells became rounded and non-adherent in PASIII-M. In contrast, PS exposure was sporadic in platelets stored in Aayusol during 9-day storage. Significant number of platelets maintain their morphology and some remain adherent at the end of storage.

PS exposure was quantitated using FACS analysis (Table 3).

**Table 3: Temporal Evaluation of PS Exposure (%) in Stored Platelets**

| **Storage Days** | **0** | **3** | **5** | **7** | **9** | |
|---|---|---|---|---|---|---|
| PASIII-M | 0.10±0.30 | 5.91±2.40 | 15.20±6.90 | 24.50±5.90 | 35.10±5.80 | n = 5 |
| Aayusol | 0.10±0.14 | 1.80±0.87 | 4.70±1.82 | 7.65±2.20 | 8.53±0.89 | n = 5 |

### Example 4

This Example shows that Aayusol can be used to preserve platelets at sub-ambient temperatures.

Platelets were stored in Aayusol for 15 days at 4 ± 1° C and 13 ± 3 °C with gentle shaking and were imaged on 0, 5, 9 and 15 days using a confocal microscope (Figure 6). The platelets maintained their morphology throughout the storage at both temperatures, albeit, with progressive retraction of ruffled edges and rounding up of the cells. Microparticles (MP) began to appear after about day 9 of storage

Forward-scattered light (FSC) is proportional to cell-surface area or size. As such, FSC provides a suitable method of detecting particles greater than a given size independent of their fluorescence and is therefore used to differentiate platelets from platelet aggregates and microparticle formation. Side-scattered light (SSC) is proportional to cell granularity or internal complexity. Figure 7 is a representative set of dot plots show platelets, aggregates, and MPs stored at 4 °C (upper panel) and at 13 °C (lower panel). There was a progressive but non-significant increase in microparticles (P; green) and aggregates (P7; blue) formation during storage, (greater at 4°C), however, the distribution of platelet population (red) at both the temperatures remained consistent during storage.

Platelets were sampled periodically at 0, 3, 5, 7, 9, 12 and 15 days and labeled with fluorescently tagged anti - CD 41a, CD 42b, CD62p (p-selectin) antibodies. PS was labeled with FITC-lactadherin. Samples were imaged with fluorescence confocal microscopy (Figure 8 upper panels) and quantitative flow cytometry (Figure 8 lower panels). Microparticles (MP) and platelets aggregates and platelets were quantitated using Forward-scattered light (FSC) and Side-scattered light (SSC) dot blots (as shown in figure 8). Platelets were robustly labeled with fluorescence markers throughout storage time. Platelet cell surface markers (CD 41 and 42b) remained intact and stable during 15-day storage at both the temperatures. Platelet activation marker (CD62p), MP formation and platelet aggregation was minimal at both the temperatures. Theses markers were more apparent in platelets stored at 4 °C than 13 °C, though not significantly different. In contrast, PS exposure markedly increased in platelets stored at 4 °C, but minimally at 13 °C. However, mitochondrial polarity assay using JC1 fluorescence dye showed that these platelets were not apoptotic but remained viable (not shown). Without being bound to theory, such partially activated platelets may rapidly home in to an actively bleeding site in a patient causing rapid hemostasis, as PS exposure is a known catalyst of the activation of coagulation cascade, hence of use in acute hemorrhagic patients.

A number of biochemical markers were also examined in platelets stored in the Aayusol solution at sub-ambient temperatures.

**Table 4: Biochemical changes in platelets stored in the Aayusol solution at sub-ambient temperatures**

| Storage (days) | | 0 | 3 | 6 | 9 | 12 | 15 |
|---|---|---|---|---|---|---|---|
| pH | 4°C | 7.31 | 7.21 | 7.23 | 7.26 | 7.29 | 7.30 |
| | 10°C | 7.24 | 7.22 | 7.25 | 7.26 | 7.25 | 7.24 |
| | 13°C | 7.24 | 7.30 | 7.29 | 7.29 | 7.27 | 7.25 |
| pCO₂ (mmHg) | 4°C | 14 | 16.8 | 14.9 | 14.1 | 12.6 | 12.7 |
| | 10°C | 16.9 | 15.2 | 13.4 | 12.3 | 11.6 | 11.4 |
| | 13°C | 15.6 | 13 | 12.6 | 11.8 | 11.6 | 11.3 |
| pO₂ (mmHg) | 4°C | 166 | 196 | 199 | 200 | 180 | 213 |
| | 10°C | 162 | 190 | 180 | 197 | 180 | 194 |
| | 13°C | 161 | 186 | 185 | 191 | 194 | 199 |
| BEeff (mmol/L) | 4°C | -19 | -21 | -21 | -21 | -20 | -20 |
| | 10°C | -20 | -21 | -21 | -22 | -22 | -22 |
| | 13°C | -21 | -20 | -20 | -21 | -22 | -22 |
| HCO₃ (mmol/L) | 4°C | 7.1 | 6.7 | 6.3 | 6.3 | 6.1 | 6.2 |
| | 10°C | 7.2 | 6.2 | 5.9 | 5.5 | 5.1 | 4.9 |
| | 13°C | 7 | 6.4 | 6.1 | 5.7 | 5.3 | 4.9 |
| Lactate (mmol/L) | 4°C | <0.3 | <0.3 | 0.39 | 0.47 | 0.52 | 0.63 |
| | 10°C | 0.35 | 0.43 | 0.89 | 1.27 | 1.61 | 1.93 |
| | 13°C | <0.3 | 0.68 | 1.11 | 1.64 | 2.06 | 2.4 |

As can be seen from Table 4, pH and base efficiency (BE eff) remained consistent throughout the storage period at all the temperatures under investigation indicating robust buffering capacity of Aayusol. As expected, there was time dependent increase in lactate demonstrating robust glucose metabolism (ascertained from the observed increase in HEP production; see figure 10, described below). Nevertheless, the increase in lactate was significantly lower than that reported in conventional ambient temperature storage, and it did not affect the pH.

NO production in platelets stored at sub-ambient temperatures was also determined. Platelets were identified by flow cytometry on the basis of their FSC/SSC distribution (i) and by surface marker discrimination (not shown). The distribution of the green fluorescence signal measured in platelet gate is depicted in Figure 9 as well as FSC and SSC. NO synthesis was visualized with the cell permeable fluorescent precursor, 4-amino-5-methylamino-2',7'- difluorofluorescein (DAF-FM) diacetate on 0, 5, 9, and 15 days by confocal microscope (upper panel). Additionally, the NO synthesis in the stored platelets was also quantified by flow cytometer from the fluorescence distribution plot of the platelets loaded with DAF-FM (lower panel). NO expression in platelets remained consistent over the storage period (left panel) with a linear decrease in NO production by about 35% at day 15 in platelets stored at 4 °C and about 10% decrease at 13 oC. This represents the first time quantitative imaging of NO has been successfully performed in stored platelets.

Platelets stored in Aayusol at 4 and 13°C for 15 days were also assessed for high energy phosphate (HEP) levels. HEP, including ATP and CP, levels were periodically assessed using established procedures. As shown in Figure 10, platelets continued to synthesize HEP at both the temperatures over 9 day storage, however, the synthesis decreased over subsequent storage; ATP by about 50% at day 15 at both temperatures; in contrast, CP decreased by about 15% in both the samples. Nevertheless, significant levels of HEP were retained by the platelets at the end of storage. Thus Aayusol efficiently supports HEP metabolism during extended storage at sub ambient temperatures.

Additional experiments showed that Platelets stored in Aayusol at sub-ambient temperatures remain functionally active upon agonist stimulation. The stored platelets were stimulated with 50 µM ADP and/or 2 µM A23187/ on 0, 1, 3, 5, 7, 9, 12 and 15 days, were stained with FITC-lactadherin (a PS probe) and Alexa 647 anti-CD62p Ab, and were analyzed by flow cytometry (Figure 11). The percentage of platelets responding to the agonist over the storage were analyzed and compared to the values obtained at baseline (0 days). Functional activation of platelets stored at 13 °C was superior to those stored at 4 °C. Nevertheless, platelets remain functionally viable at both temperatures during extended storage.

These results clearly indicate that platelets can be stored in viable and functionally active form at sub-ambient temperatures in Aayusol, a first.

## Claims

1. A composition for preserving platelets comprising: a physiological salt solution, glutathione, ascorbic acid, adenosine, dichloroacetate, and less than 1 µM calcium ions wherein the composition has a pH of 7 to 7.7.

2. The composition of claim 1, comprising 0.001-0.5 mM dichloroacetate.

3. The composition of claim 1, wherein the physiological salt solution comprises one or more salts selected from the group consisting of potassium chloride, potassium phosphate, magnesium chloride, magnesium sulfate, sodium chloride, sodium bicarbonate and sodium phosphate.

4. The composition of any one of claims 1-3, comprising 4-5 mM of potassium chloride, 0.3-0.5 mM of potassium phosphate, 0.4-0.6 mM of magnesium chloride, 0.4-0.6 mM of magnesium sulfate, 100-115 mM sodium chloride, 4-6 mM of sodium bicarbonate, and 10-40 mM sodium phosphate.

5. The composition of any one of claims 1-4, further comprising 2.5-5 mM creatine.

6. The composition of any one of claims 1-5, further comprising 0.5-2 mM orotic acid.

7. The composition of any one of claims 1-6, further comprising 11-25 mM of a sugar, wherein the sugar is glucose or dextrose.

8. The composition of any one of claims 1-7, further comprising 2-10 mM arginine.

9. The composition of any one of claims 1-8, further comprising 0.001-10 mM malic acid.

10. The composition of any one of claim 1-9, further comprising 0.001-10 mM citrulline malate.

11. The composition of any one of claims 1-10, further comprising 5-10 mM carnosine.

12. The composition of any one of claims 1-11, further comprising 5-10 mM carnitine.

13. The composition of any one of claims 1-12, wherein the composition does not contain one or more of calcium ions or insulin.

14. The composition of any one of claims 1-13, further comprising a calcium chelator.

15. The composition of claim 14, wherein the calcium chelator is selected from the group consisting of ethylenediaminetetraacetic acid (EDTA), ethyleneglycoltetraacetic acid (EGTA), diethylenetriaminepentaacetate (DTPA), hydroxyethylethylenediaminetriacetic acid (HEEDTA), diaminocyclohexanetetraacetic acid (CDTA), 1,2-bis(2-aminophenoxy)ethane-N,N,N',N'-tetraacetic acid (BAPTA), and citric acid.

## Patentansprüche

1. Zusammensetzung zur Konservierung von Blutplättchen, umfassend: eine physiologische Salzlösung, Glutathion, Ascorbinsäure, Adenosin, Dichloracetat und weniger als 1 µM Calciumionen, wobei die Zusammensetzung einen pH-Wert von 7 bis 7,7 hat.

2. Zusammensetzung nach Anspruch 1, umfassend 0,001-0,5 mM Dichloracetat.

3. Zusammensetzung nach Anspruch 1, wobei die physiologische Salzlösung ein oder mehrere Salze umfasst, ausgewählt aus der Gruppe bestehend aus Kaliumchlorid, Kaliumphosphat, Magnesiumchlorid, Magnesiumsulfat, Natriumchlorid, Natriumbicarbonat und Natriumphosphat.

4. Zusammensetzung nach einem der Ansprüche 1 bis 3, umfassend 4 bis 5 mM Kaliumchlorid, 0,3 bis 0,5 mM Kaliumphosphat, 0,4 bis 0,6 mM Magnesiumchlorid, 0,4 bis 0,6 mM Magnesiumsulfat, 100 bis 115 mM Natriumchlorid, 4 bis 6 mM Natriumbicarbonat und 10 bis 40 mM Natriumphosphat.

5. Zusammensetzung nach einem der Ansprüche 1 bis 4, ferner umfassend 2,5-5 mM Kreatin.

6. Zusammensetzung nach einem der Ansprüche 1 bis 5, ferner umfassend 0,5-2 mM Orotsäure.

7. Zusammensetzung nach einem der Ansprüche 1 bis 6, ferner umfassend 11-25 mM eines Zuckers, wobei der Zucker Glucose oder Dextrose ist.

8. Zusammensetzung nach einem der Ansprüche 1 bis 7, ferner umfassend 2-10 mM Arginin enthält.

9. Zusammensetzung nach einem der Ansprüche 1 bis 8, ferner umfassend 0,001-10 mM Apfelsäure.

10. Zusammensetzung nach einem der Ansprüche 1 bis 9, ferner umfassend 0,001-10 mM Citrullinmalat.

11. Zusammensetzung nach einem der Ansprüche 1 bis 10, ferner umfassend 5-10 mM Carnosin.

12. Zusammensetzung nach einem der Ansprüche 1 bis 11, ferner umfassend 5-10 mM Carnitin enthält.

13. Zusammensetzung nach einem der Ansprüche 1 bis 12, wobei die Zusammensetzung nicht eines oder mehrere Kalziumionen oder Insulin enthält.

14. Zusammensetzung nach einem der Ansprüche 1 bis 13, ferner umfassend einen Calciumchelator.

15. Zusammensetzung nach Anspruch 14, wobei der Calciumchelator ausgewählt ist aus der Gruppe bestehend aus Ethylendiamintetraessigsäure (EDTA), Ethylenglykoltetraessigsäure (EGTA), Diethylentriaminpentaacetat (DTPA), Hydroxyethylethylendiamintetraessigsäure (HEEDTA), Diaminocyclohexantetraessigsäure (CDTA), 1,2-Bis(2-aminophenoxy)ethan-N,N,N',N'-tetraessigsäure (BAPTA) und Zitronensäure.

## Revendications

1. Composition pour conserver les plaquettes comprenant : une solution saline physiologique, du glutathion, de l'acide ascorbique, de l'adénosine, du dichloroacétate, et moins de 1 µM d'ions calcium, la composition ayant un pH de 7 à 7,7.

2. Composition selon la revendication 1, comprenant de 0,001 à 0,5 mM de dichloroacétate.

3. Composition selon la revendication 1, dans laquelle la solution saline physiologique comprend un ou plusieurs sels choisis dans le groupe constitué du chlorure de potassium, du phosphate de potassium, du chlorure de magnésium, du sulfate de magnésium, du chlorure de sodium, du bicarbonate de sodium et du phosphate de sodium.

4. Composition selon l'une quelconque des revendications 1 à 3, comprenant 4 à 5 mM de chlorure de potassium, 0,3 à 0,5 mM de phosphate de potassium, 0,4 à 0,6 mM de chlorure de magnésium, 0,4 à 0,6 mM de sulfate de magnésium, 100 à 115 mM de chlorure de sodium, 4 à 6 mM de bicarbonate de sodium et 10 à 40 mM de phosphate de sodium.

5. Composition selon l'une quelconque des revendications 1 à 4, comprenant en outre 2,5 à 5 mM de créatine.

6. Composition selon l'une quelconque des revendications 1 à 5, comprenant en outre 0,5 à 2 mM d'acide orotique.

7. Composition selon l'une quelconque des revendications 1 à 6, comprenant en outre 11 à 25 mM d'un sucre, le sucre étant le glucose ou le dextrose.

8. Composition selon l'une quelconque des revendications 1 à 7, comprenant en outre 2 à 10 mM d'arginine.

9. Composition selon l'une quelconque des revendications 1 à 8, comprenant en outre 0,001 à 10 mM d'acide malique.

10. Composition selon l'une quelconque des revendications 1 à 9, comprenant en outre 0,001 à 10 mM de malate de citrulline.

11. Composition selon l'une quelconque des revendications 1 à 10, comprenant en outre 5 à 10 mM de carnosine.

12. Composition selon l'une quelconque des revendications 1 à 11, comprenant en outre 5 à 10 mM de carnitine.

13. Composition selon l'une quelconque des revendications 1 à 12, la composition ne contenant pas l'un ou plusieurs parmi des ions calcium ou de l'insuline.

14. Composition selon l'une quelconque des revendications 1 à 13, comprenant en outre un chélateur de calcium.

15. Composition selon la revendication 14, dans laquelle le chélateur de calcium est choisi dans le groupe constitué de l'acide éthylènediaminetétraacétique (EDTA), l'acide éthylèneglycoltétraacétique (EGTA), le pentaacétate de diéthylènetriamine (DTPA), l'acide hydroxyéthyléthylènediaminetétraacétique (HEEDTA), l'acide diaminocyclohexanetétraacétique (CDTA), l'acide 1,2-bis(2-aminophénoxy)éthane-N,N,N',N'-tétraacétique (BAPTA) et l'acide citrique.
